(19) **Europäisches Patentamt / European Patent Office / Office européen des brevets**

(11) **EP 3 777 562 B1**

(12) # FASCICULE DE BREVET EUROPEEN

(45) Date de publication et mention de la délivrance du brevet:
**06.07.2022 Bulletin 2022/27**

(21) Numéro de dépôt: **20188478.0**

(22) Date de dépôt: **29.07.2020**

(51) Classification Internationale des Brevets (IPC):
**A23L 21/20** *(2016.01)*    **A61K 35/644** *(2015.01)*
**A23L 29/00** *(2016.01)*    **A23L 2/52** *(2006.01)*

(52) Classification Coopérative des Brevets (CPC):
(C-Sets disponibles)
**A23L 21/20; A23L 2/52; A23L 29/03; A61K 35/644;**
A23L 19/09; A23V 2002/00                    (Cont.)

(54) **PRÉPARATION À BASE DE PULPE DE FRUIT DE BAOBAB POUR STABILISER UNE PRÉPARATION COMPRENANT DE LA GELÉE ROYALE**

BAOBAB-FRUCHTFLEISCHZUBEREITUNG ZUR STABILISIERUNG EINER ZUBEREITUNG MIT GELÉE ROYALE

PREPARATION USING BAOBAB FRUIT PULP FOR STABILISING A PREPARATION CONTAINING ROYAL JELLY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.08.2019 FR 1908932**

(43) Date de publication de la demande:
**17.02.2021 Bulletin 2021/07**

(73) Titulaire: **Laboratoires Arkopharma**
**06510 Carros Cedex (FR)**

(72) Inventeurs:
• **BONNOTTE, Martine**
  **06200 Nice (FR)**
• **ROBINET, Philippe**
  **06580 Pégomas (FR)**

(74) Mandataire: **Murgitroyd & Company**
**Murgitroyd House**
**165-169 Scotland Street**
**Glasgow G5 8PL (GB)**

(56) Documents cités:
**WO-A1-2004/089431     CN-A- 1 076 362**
**JP-A- 2002 176 936     US-A1- 2015 265 665**

• **DATABASE GNPD [Online] MINTEL; 5 avril 2013 (2013-04-05), anonymous: "Boost", XP055634373, extrait de www.gnpd.com Database accession no. 2039162**

(52) Classification Coopérative des Brevets (CPC):
(Cont.)

C-Sets
A23V 2002/00, A23V 2200/244, A23V 2250/21,
A23V 2300/24

(52) Classification Coopérative des Brevets (CPC):
(Cont.)

C-Sets

**Description**

**Domaine technique**

La présente invention concerne le domaine de la gelée royale et des compositions comprenant de la gelée royale, à usage humain ou animal, notamment sous forme de compléments alimentaires.

**Etat de la technique**

La gelée royale est un produit issu de la ruche très connu et extrêmement populaire. La gelée royale représente la nourriture produite par les abeilles *(Apis mellifera* L.), destinée à la reine dont c'est la nourriture exclusive, ce qui permettrait à cette dernière notamment d'avoir une espérance de vie moyenne environ quarante-cinq fois supérieure à celle des abeilles ouvrières. La gelée royale fait également partie de l'alimentation des larves d'abeilles jusqu'au troisième jour de leur existence, ce qui leur permet de croître normalement.

Produite par le système glandulaire céphalique des abeilles ouvrières, la gelée royale a un goût à la fois sucré et acide.

La gelée royale est une source exceptionnelle d'acides aminés, de vitamine B (indispensable au bon fonctionnement du métabolisme, au système nerveux et à la croissance des cellules) et d'oligo-éléments. Il s'agit donc d'une substance de premier choix produite par les abeilles (« produit de la ruche ») tout comme le miel, la propolis, le pain d'abeille ou le pollen.

La gelée royale peut se consommer simplement sous forme fraîche (par exemple conditionnée dans un pot obturable par un couvercle), par exemple en laissant fondre une petite cuillère de gelée royale sous la langue, de préférence le matin au réveil. Toutefois, la gelée royale « fraîche » impose des conditions drastiques de stockage (stockage dans un contenant, de préférence le contenant originel, correctement obturé dans un lieu sec et protégé de la lumière à une température comprise entre 0°C et 4°C), afin d'éviter des problèmes de stabilité et/ou des problèmes sanitaires liés à l'apparition et à la croissance de micro-organismes potentiellement pathogènes au sein de ladite gelée royale.

Afin d'assurer une bonne stabilité dans le temps de la gelée royale à température ambiante, tout en évitant ou limitant le développement de micro-organismes potentiellement pathogènes, des stratégies ont été développées dans l'art antérieur. Ces stratégies s'articulent autour de deux axes principaux :

(i) ajouter un ou plusieurs conservateur(s) au sein de la préparation comprenant la gelée royale, ou
(ii) soumettre la gelée royale à un procédé thermique de décontamination, par exemple de type stérilisation.

Concernant les produits à base de gelée royale mis au point en suivant l'axe (i) supra, il est possible de citer le produit « GRANIONS® GELEE ROYALE 1500 mg stick 12 ml » de la société GRANIONS®, dont la composition est présentée dans le tableau 1 infra :

Tableau 1

| Composition du produit «GRANIONS® - GELEE ROYALE 1500 mg stick 12 ml » de la société GRANIONS® | |
| --- | --- |
| Eau | |
| Gelée royale | 1500 mg |
| Miel | 1200 mg |
| Fructose | 600 mg |
| Acide malique | |
| Extrait acerola | 50.4 mg |
| Sorbate de potassium, benzoate de sodium | |
| Liquide jaune limpide acide et piquant | |
| Ph | 3.37 |
| Densité | 1.064 |
| Brix | 17 |

Ce produit est obtenu sans traitement thermique préalable mais comprend, en tant que conservateur, du sorbate de potassium et du benzoate de sodium. Ceci représente un inconvénient majeur, dans la mesure où il convient, dans la mesure du possible, de s'affranchir de l'utilisation de tels conservateurs pour des raisons sanitaires évidentes et bien connues. En effet, le benzoate de sodium (E211) est classé « rouge - A EVITER » par un site de référence, à savoir le site « Que Choisir ». Le benzoate de sodium est un sel de sodium de l'acide benzoïque (E210), lui-même dérivé du benzène. Les remarques formulées pour l'acide benzoïque s'appliquent par extension au benzoate de sodium. Ainsi, la dose journalière admissible (DJA) peut être dépassée, en particulier chez les enfants forts consommateurs d'aliments vecteurs (comme les boissons aromatisées). Des réactions d'hypersensibilité et d'allergie ont aussi été relevées (asthme, prurit, rougeurs). Ce conservateur est enfin soupçonné de favoriser l'hyperactivité chez les enfants, seul ou en combinaison avec des colorants azoïques (cf. https://www.quechoisir.org/comparatif-additifs-alimentairesn56877/e211-benzoate-de-sodium-p223253/#tab-description). Des publications récentes et régulières font aussi état de liens potentiels avec des déficits cognitifs notamment à court terme et avec l'obésité infantile, notamment dus à la consommation de boissons sucrées qui en contiennent (comme, par exemple, des sirops tels que le sirop de grenadine). Les DJA sont couramment dépassées par les enfants notamment par la consommation de boissons (sodas, sirops).

Concernant le sorbate de potassium (E202), une controverse existe autour d'une mutagénicité potentielle des sorbates en association avec des nitrites.

A titre de produits à base de gelée royale obtenus en mettant en œuvre l'axe (ii) supra, il est possible de citer les produits suivants :

a) le produit « STIM® Royal » de la société Nutreov dont la composition est présentée dans le tableau 2 infra,

b) le produit « GELEE ROYALE BIO 2000 mg » (en ampoule de 15 ml) de la société FORTE PHARMA dont la composition est présentée dans le tableau 3 infra,

c) le produit «GELEE ROYALE BIO 1800 mg » (en ampoule de 10 ml) de la société NUTRISANTE dont la composition est présentée dans le tableau 4 infra, et

d) le produit « GELEE ROYALE BIO » (en ampoule de 10 ml) de la société NAT & FORM dont la composition est présentée dans le tableau 5 infra,

Tableau 2

| Composition du produit « STIM® Royal » de la société Nutreov. |
|---|
| Pour une ampoule de 10 ml: Eau, Miel, Gelée Royale 1500 mg, Extraits secs sur Maltodextrine: Acérola Baie Malpighia Punicifolia L. (soit Vitamine C 16 mg, 20% des AJR*), Eleuthérocoque racine Eleutherococcus Senticosus 300 mg, Jus d'Argousier Hippophae Rhamnoides L. 250 mg, Solution de Propolis 100 mg (soit Propolis 14 mg), Arôme naturel Orange, Vitamines: B3 (Nicotinamide 4.8 mg, soit 30% des AJR*) B9 (Acide Folique 60 $\mu$g, 30% des AJR*), Edulcorant: Glycosides de Stéviol |

Tableau 3

| Composition du produit « GELEE ROYALE BIO 2000 mg » (en ampoule de 15 ml) de la société FORTE PHARMA | |
|---|---|
| Eau purifiée | |
| Gelé royale bio | 2000 mg |
| Miel bio | 2000 mg |
| Liquide gélifié avec gelée en fins morceaux contenant des dépôts après agitation, sucré et peu piquant, gélifié. | |
| pH | 4.00 |
| Densité | 1.055 |
| Brix 11° | |

Tableau 4

| Composition du produit « GELEE ROYALE BIO 1800 mg » (en ampoule de 10 ml) de la société NUTRISANTE | |
|---|---|
| Eau purifiée | |
| Sirop d'agave bio | 3500 mg |
| Gelé royale bio | 1800 mg |
| Jus d'orange concentré bio | |
| Liquide gélifié marron gout sucré et un peu piquant | |
| pH | 3.93 |

(suite)

| Composition du produit « GELEE ROYALE BIO 1800 mg » (en ampoule de 10 ml) de la société NUTRISANTE | |
|---|---|
| Densité | 1.145 |
| Brix | 34° |

Tableau 5

| Composition du produit « GELEE ROYALE BIO » (en ampoule de 10 ml) de la société NAT & FORM | |
|---|---|
| Eau | |
| Miel bio | 5000 mg |
| Gelé royale bio | 1500 mg |
| Produit gélifié marron avec liquide surnageant | |
| pH | 3.95 |
| Densité | 1.168 |
| Brix | 38° |

Toutefois, les inventeurs ont découvert que l'ensemble des produits à base de gelée royale présentés aux points a)-d) présentent un problème de gélification rendant mal aisé l'écoulement du produit hors de l'ampoule en verre à extrémités cassables (ampoule en verre « à deux pointes »), après rupture desdites extrémités. Il a même été constaté que, pour certains de ces produits, il était nécessaire de souffler par un des orifices de l'ampoule en verre, après rupture des extrémités cassables pour permettre l'écoulement du produit à base de gelée royale. Ceci représente bien évidemment une contrainte importante pour le consommateur, notamment susceptible de diminuer la quantité de gelée royale ingérée (eu égard à la quantité de gelée royale gélifiée restant dans l'ampoule en verre après rupture des deux extrémités cassables de cette ampoule) mais également un problème sanitaire si le consommateur est contraint de porter l'ampoule en verre cassé à sa bouche, de souffler en son sein afin d'espérer l'écoulement du produit à base de gelée royale contenu au sein de l'ampoule (risques de blessures au niveau de la cavité buccale du consommateur).

En tant qu'un des leaders sur le secteur, la demanderesse possède elle aussi une gamme historique de produits à base de gelée royale ; produits regroupés sous le nom de marque ARKOROYAL®. Ces produits contiennent entre 500 mg et 1500 mg de gelée royale. La gamme de produits ARKOROYAL® comprend des formulations en ampoule classique de 10 ml (ampoule en verre à extrémités cassables, communément dénommée « ampoule à deux pointes ») et en UNICADOSE® (ampoules en plastique [en polypropylène] à fond plat et à extrémité cassable) de 15 ml. Au sein de cette gamme de produits ARKOROYAL®figure, par exemple, le produit ARKOROYAL® ROYAL'FRUITS. Ce produit, garanti sans composé de synthèse (« ingrédient chimique ») ni conservateur, comprend, outre 1000 mg de gelée royale, de qualité premium, trois « super » fruits (goji *(Lycium barbarum L.),* grenade *(Punica granatum L.)* et baobab *(Adansonia digitata L.)).* Plus précisément, ce produit comprend 600 mg de jus concentré de baie de goji, 200 mg de jus concentré de grenade et 15 mg de poudre de pulpe de fruit de baobab. Tel qu'indiqué sur le site Internet correspondant, https://www.arkopharma.com/fr-FR/arkoroyal-royalfruits, la pulpe de fruit de baobab est considérée comme riche en éléments nutritifs. Ce produit est indiqué pour reconstituer l'organisme et se maintenir en pleine santé.

Afin de ne pas inclure de conservateurs au sein des formulations de la gamme ARKOROYAL®, pour les raisons exposées précédemment, la demanderesse a également opté pour un procédé thermique de décontamination/désinfection desdites formulations afin d'en optimiser/préserver la qualité microbiologique. Ce faisant, et également confrontée au problème de gélification de la gelée royale lorsque celle-ci est soumise audit procédé thermique de décontamination, elle s'est vue contrainte de développer un procédé de préparation spécifique, alliant étape(s) de chauffage, étape(s) de refroidissement et étape(s) de cisaillement. Si ce procédé s'avère globalement efficace, il n'en reste pas moins complexe à mettre en œuvre, onéreux, et requiert la mise en œuvre d'appareillage spécifique/complexe.

Au regard de ce qui précède, il existe donc réellement un besoin visant à stabiliser efficacement la gelée royale (et de préférence tous types de gelée royale, indépendamment de leur origine) et les compositions la comprenant, en particulier lorsque la gelée royale ou lesdites compositions sont soumises à un traitement thermique de type pasteurisation ou stérilisation, tout en :

i) préservant l'efficacité, la qualité (par exemple la qualité nutritive), les propriétés physico-chimiques et/ou les propriétés organoleptiques de la gelée royale,
ii) évitant, pour ce faire, la nécessité de développer un procédé de préparation complexe à mettre en œuvre, onéreux, et/ou requérant la mise en œuvre d'appareillage spécifique/complexe,

iii) avantageusement s'affranchissant de l'utilisation de conservateur(s), et également de préférence en évitant de recourir à des composés de synthèse.

Cherchant à répondre à ce besoin, les inventeurs ont découvert, de manière surprenante, que la poudre de pulpe de fruit de baobab présente au sein du produit ARKOROYAL® ROYAL'FRUITS susvisé permettait, outre les propriétés extrinsèques présentées supra (en particulier les propriétés nutritives), d'obtenir des propriétés intrinsèques de première importance concernant la stabilisation de la gelée royale, à savoir non seulement la stabilisation dans le temps de celle-ci mais également - et surtout - en matière de prévention, de limitation et/ou d'inhibition du phénomène de gélification se produisant lors du procédé thermique utilisé pour la production de ce produit ARKOROYAL® ROYAL'FRUITS.

JP2002176936(A) propose l'utilisation d'amidon et d'un procédé thermique pour fournir un produit liquide stabilisé à base de gelée royale.

## Exposé de l'invention

Par conséquent, l'invention a pour objet l'utilisation d'au moins une préparation à base de pulpe de fruit de baobab (de préférence de baobab africain, *Adansonia digitata* L.) pour stabiliser une préparation comprenant de la gelée royale (par exemple une préparation à base de gelée royale).

Selon un mode de réalisation préféré, au sens de la présente invention, l'action de stabiliser comprend, consiste essentiellement à, ou consiste à :

- stabiliser dans le temps ladite préparation comprenant de la gelée royale, et/ou
- prévenir, limiter et/ou inhiber (totalement ou partiellement) la gélification de ladite préparation comprenant de la gelée royale, de préférence lorsque ladite préparation comprenant de la gelée royale est soumise à un traitement thermique tel qu'une pasteurisation ou une stérilisation, préférablement soumise à une pasteurisation, avantageusement à une température comprise entre environ 60°C et environ 99°C, et de manière préférée à une température comprise entre environ 75°C et environ 95°C (et de manière particulièrement préférée à une température comprise entre environ 85°C et environ 95°C).

Selon un mode de réalisation, le susdit traitement thermique est un traitement thermique de type pasteurisation ou stérilisation, préférablement de type pasteurisation, avantageusement réalisé à une température comprise entre environ 60°C et environ 99°C, et de manière préférée à une température comprise entre environ 75°C et environ 95°C (et de manière particulièrement préférée à une température comprise entre environ 85°C et environ 95°C).

En fonction du besoin, la durée du traitement thermique (par exemple de la pasteurisation) peut varier d'une seconde ou de quelques secondes (par exemple dans le cas d'une « pasteurisation flash ») à environ 90 minutes.

Le traitement thermique susvisé est réalisé, en particulier, afin d'optimiser la qualité et/ou de protéger la susdite préparation d'un point de vue microbiologique.

Selon un mode de réalisation préféré de l'invention, le traitement thermique de la préparation comprenant de la gelée royale est une pasteurisation. En effet, la demanderesse a découvert que cette technique présentait notamment l'avantage d'être efficace pour l'optimisation de la qualité microbiologique de ladite préparation, tout en :

- préservant la qualité de la gelée royale (en particulier sa qualité nutritive), ses caractéristiques physico-chimiques, et également, de manière avantageuse, ses propriétés organoleptiques, et en
- étant relativement peu onéreux.

Selon un aspect préféré de l'invention, le susdit traitement thermique est une pasteurisation dont les paramètres de température et de durée sont définis comme suit :

- une température comprise entre environ 60°C et environ 99°C (par exemple entre 60°C et 99°C), préférablement comprise entre environ 75°C et environ 95°C (par exemple entre 75°C et 95°C), avantageusement comprise entre environ 85°C et environ 95°C (par exemple entre 85°C et 95°C), et
- une durée comprise entre environ 30 minutes et environ 90 minutes (par exemple entre 30 et 90 minutes), préférablement comprise entre environ 45 minutes et environ 75 minutes (par exemple entre 45 et 75 minutes), avantageusement comprise entre environ 55 minutes et environ 65 minutes (par exemple entre 55 et 65 minutes) ; de manière préférée ladite durée étant d'environ 60 minutes (par exemple de 60 minutes).

Avantageusement, selon cet aspect préféré de l'invention, les paramètres de température et de durée de la pasteurisation sont définis comme suit :

- une température comprise entre environ 85°C et environ 95°C (par exemple entre 85°C et 95°C), et
- une durée comprise entre environ 55 et environ 65 minutes (par exemple entre 55 et 65 minutes).

De préférence, la préparation comprenant de la gelée royale est dépourvue de conservateur et avantageusement également dépourvue de composé de synthèse. Ceci représente un avantage significatif eu égard aux problèmes - et potentiels problèmes - sanitaires liés à l'emploi de conservateur(s) (cf. supra). Avantageusement, la préparation à base de pulpe de fruit de baobab est également dépourvue de conservateur (et préférablement de composé de synthèse), pour les raisons exposées précédemment.

Selon un mode de réalisation, la gelée royale est sélectionnée parmi la gelée royale fraîche, la gelée royale décongelée, la gelée royale lyophilisée, la gelée royale atomisée. En effet, et tel que démontré dans l'exemple 1.1 infra, la présente invention trouve à s'appliquer à tous types et à toutes sources/origines de gelée royale.

Selon un mode de réalisation, la préparation comprenant de la gelée royale consiste en :

i) la gelée royale, ou

ii) une préparation comprenant, consistant essentiellement en, ou consistant en, un mélange de gelée royale et d'eau, de préférence dans un rapport en masse gelée royale/eau compris entre 5/200 et 1/2, préférablement entre 5/100 et 2/5, avantageusement entre 2/25 et 3/10 ; de préférence ladite préparation ii) étant sous forme de solution buvable, avantageusement adaptée pour être conditionnée dans un contenant à usage unique clos, de préférence de manière hermétique, tel qu'un sachet unidose, un stick unidose, une flaconnette, une ampoule bouteille unidose, ou une ampoule, de préférence une ampoule, préférablement une ampoule à extrémité(s) cassable(s), avantageusement une ampoule à extrémités cassables telle qu'une ampoule en verre, par exemple d'une contenance de 10 ml.

Selon un mode de réalisation, ladite au moins une préparation à base de pulpe de fruit de baobab est sélectionnée parmi : poudre de pulpe de fruit de baobab et/ou extrait de pulpe de fruit de baobab ; ladite au moins une préparation à base de pulpe de fruit de baobab étant avantageusement de la poudre de pulpe de fruit de baobab. Selon un mode de réalisation particulier, l'on utilise une pluralité de préparations différentes à base de pulpe de fruit de baobab, par exemple une poudre de pulpe de fruit de baobab et un extrait de pulpe de fruit de baobab.

La susdite au moins une préparation à base de pulpe de fruit de baobab est utilisée, dans une composition comprenant une préparation comprenant de la gelée royale, en quantité efficace pour stabiliser ladite préparation comprenant de la gelée royale. Selon un mode de réalisation, la masse de ladite au moins une préparation à base de pulpe de fruit de baobab représente au moins 0,5% (par exemple plus de 0,5%), de préférence au moins 1%, préférablement au moins 1,3%, avantageusement au moins 1,5% (par exemple plus de 1,5%), de manière préférée au moins 1,8%, et de manière particulièrement préférée au moins 2%, de la masse de gelée royale.

Selon un mode de réalisation, la masse de ladite au moins une préparation à base de pulpe de fruit de baobab représente au maximum 10% (par exemple moins de 10%), de préférence au maximum 7%, avantageusement au maximum 5%, de la masse de gelée royale.

Selon un mode de réalisation, au moins x% de ladite au moins une préparation à base de pulpe de fruit de baobab, défini(s) par rapport à la masse de gelée royale, est/sont mélangé(s) à la préparation comprenant de la gelée royale pour atteindre une viscosité cinématique critique prédéterminée y, à partir de laquelle l'écoulement du mélange est optimal, et dans laquelle ledit/lesdits x% est/sont déterminé(s) par la relation suivante :

$$x\% = \frac{y + 14,222}{22,248}$$

Selon un mode de réalisation, ladite préparation comprenant de la gelée royale comprend, consiste essentiellement en, ou consiste en :

- 1000 mg de gelée royale et ladite au moins une préparation à base de pulpe de fruit de baobab est utilisée à raison d'au moins 0,5% en masse (par exemple plus de 0,5% en masse), de préférence d'au moins 1% en masse, par rapport à la masse de la gelée royale, ou
- 1500 mg de gelée royale et ladite au moins une préparation à base de pulpe de fruit de baobab est utilisée à raison d'au moins 0,5% en masse (par exemple plus de 0,5% en masse), de préférence d'au moins 1% en masse, préférablement d'au moins 1,33% en masse, par rapport à la masse de la gelée royale, ou
- 2000 mg de gelée royale et ladite au moins une préparation à base de pulpe de fruit de baobab est utilisée à raison d'au moins 1% en masse (par exemple plus de 1% en masse), de préférence d'au moins 1,5% en masse, préférablement d'au moins 2% en masse, et avantageusement d'au moins 2,5% en masse, par rapport à la masse de la gelée royale.

Un autre objet de l'invention concerne une composition comprenant, consistant essentiellement en, ou consistant en :

i) au moins une préparation à base de pulpe de fruit de baobab telle que définie précédemment, et

ii.1) une préparation comprenant de la gelée royale telle que définie précédemment, la masse de ladite au moins une préparation à base de pulpe de fruit de baobab représentant plus de 1,5%, de préférence au moins 1,8%, préférablement au moins 2%, avantageusement au moins 3%, de la masse de gelée royale, ou

ii.2) une préparation comprenant de la gelée royale comprenant, consistant essentiellement en, ou consistant en :

- 1500 mg de gelée royale, et ladite au moins une préparation à base de pulpe de fruit de baobab représentant au moins 0,5% en masse (par exemple plus de 0,5% en masse), de préférence au moins 1% en masse, préférablement au moins 1,33% en masse, par rapport à la masse de la gelée royale, ou
- 2000 mg de gelée royale, et ladite au moins une préparation à base de pulpe de fruit de baobab représentant au moins 1% en masse (par exemple plus de 1% en masse), de préférence au moins 1,5% en masse, préférablement au moins 2% en masse, et avantageusement au moins 2,5% en masse, par rapport à la masse de la gelée royale.

de préférence ladite composition étant dépourvue de conservateur et avantageusement également dépourvue de composé de synthèse.

Cette composition bénéficie non seulement des propriétés nutritives de la poudre de pulpe de fruit de baobab mais surtout des propriétés inhérentes à celle-ci, non seulement en matière de stabilisation dans le temps de la gelée royale contenue au sein de ladite préparation mais également - et surtout - en matière de prévention, de limitation et/ou d'inhibition du phénomène de gélification se produisant lors du procédé thermique utilisé afin de protéger la susdite composition d'un point de vue microbiologique.

Selon un mode de réalisation, la masse de ladite au moins une préparation à base de pulpe de fruit de baobab i) représente au maximum 10% (par exemple moins de 10%), de préférence au maximum 7%, avantageusement au maximum 5%, de la masse de gelée royale.

Selon un mode de réalisation préféré, ladite composition selon l'invention comprend, consiste essentiellement en, ou consiste en :

- environ 1500 mg de gelée royale (par exemple 1500 mg de gelée royale), et
- entre environ 30 mg et environ 70 mg (par exemple entre 30 et 70 mg), de préférence entre environ 40 mg et environ 60 mg (par exemple entre 40 et 60 mg), préférablement entre environ 45 mg et environ 55 mg (par exemple entre 45 et 55 mg), avantageusement environ 50 mg (par exemple 50 mg), de ladite au moins une préparation à base de pulpe de fruit de baobab.

La composition selon l'invention est sous forme liquide, de préférence administrable par voie orale à un individu humain ou animal (de préférence humain), et avantageusement sous forme buvable.

L'invention concerne également un complément alimentaire (à usage humain ou animal, de préférence à usage humain), un dispositif médical ou un ADDFMS (cf. définition infra), comprenant la composition selon l'invention.

Un autre objet de l'invention concerne la composition selon l'invention pour son utilisation :

- en tant qu'immunostimulant (en particulier pour renforcer l'immunité, par exemple pour soutenir les convalescents),
- en tant qu'immuno-modulateur,
- en tant qu'anti-inflammatoire,
- en tant antiseptique (antimicrobien, notamment antibactérien et/ou et antiviral),
- en tant qu'anti-infectieux,
- en tant que fortifiant,
- en tant que tonifiant,
- en tant qu'adaptogène,
- en tant qu'anti-hypercholestérolémiant (notamment pour équilibrer les taux sanguins lipidiques),
- en tant qu'hypoglycémiant (notamment pour équilibrer la glycémie mais également pour aider au traitement du diabète),
- en tant qu'anti-oxydant,
- pour réduire la fatigue physique,
- pour réduire la fatigue intellectuelle,
- pour prévenir et/ou lutter contre le stress,

- pour favoriser la guérison des ulcères du pied,
- pour favoriser l'équilibre hormonal et/ou prévenir les déséquilibres hormonaux,
- pour induire un effet oestrogénique,
- pour réduire/atténuer les symptômes de la ménopause, et/ou
- pour prévenir, réguler et/ou traiter l'ostéoporose (en particulier pour atténuer l'ostéoporose).

L'invention a également pour objet un contenant à usage unique clos, de préférence de manière hermétique, tel qu'un sachet unidose, un stick unidose, une flaconnette, ou une ampoule à extrémité(s) cassable(s), ledit contenant comprenant la composition selon l'invention, ledit contenant étant de préférence une ampoule à extrémités cassables en plastique ou en verre, de préférence en verre, par exemple d'une contenance de 10 ou 15 ml.

L'invention a également pour objet un procédé d'obtention d'une composition selon l'invention dans laquelle la préparation comprenant de la gelée royale est une préparation comprenant, consistant essentiellement en, ou consistant en, un mélange de gelée royale et d'eau (de préférence dans les proportions définies précédemment), ledit procédé comprenant les étapes suivantes :

- introduire une quantité d'eau froide (à savoir non chauffée ; par exemple à température ambiante) appropriée dans un mélangeur sous agitation,
- toujours sous agitation, introduire la gelée royale et la préparation à base de pulpe de fruit de baobab telle que définie précédemment (par exemple la poudre de pulpe de fruit de baobab) dans le mélangeur, en quantités appropriées, et
- maintenir l'agitation jusqu'à obtention d'un mélange homogène, celui-ci représentant la composition selon l'invention.

Un autre objet de l'invention concerne un procédé thermique de stabilisation (ou de fluidification) et de décontamination d'une préparation comprenant de la gelée royale telle que définie précédemment (et en particulier de la gelée royale comprise dans ladite préparation), ledit procédé comprenant les étapes suivantes :

a) obtenir ladite préparation comprenant de la gelée royale, de préférence comprenant, consistant essentiellement en, ou consistant en, un mélange de gelée royale et d'eau, de préférence dans un rapport en masse gelée royale/eau compris entre 5/200 et 1/2, préférablement entre 5/100 et 2/5, avantageusement entre 2/25 et 3/10,

b) introduire ladite préparation au sein d'un récipient, de préférence obturable, avantageusement de manière hermétique, ledit récipient étant adapté pour réaliser un traitement thermique de décontamination tel qu'un traitement de type pasteurisation ou stérilisation, préférablement de type pasteurisation,

c) à l'étape b), ou de préférence avant ladite étape b) (par exemple à l'étape a)), mélanger ladite préparation comprenant de la gelée royale avec ladite au moins une préparation à base de pulpe de fruit de baobab telle que définie précédemment,

d) postérieurement à l'étape b), effectuer, au sein dudit récipient, un traitement thermique de type pasteurisation ou stérilisation, de préférence de type pasteurisation, avantageusement à une température comprise entre environ 60°C et environ 99°C, préférablement à une température comprise entre environ 75°C et environ 95°C.

Selon un mode de réalisation, lorsque ladite préparation comprenant de la gelée royale est une préparation comprenant un mélange de gelée royale et d'eau, ladite au moins une préparation à base de pulpe de fruit de baobab est, de préférence, introduite dans l'eau de ladite préparation comprenant de la gelée royale concomitamment - ou simultanément - à la gelée royale (à l'étape a)).

De préférence, et tel qu'indiqué précédemment, la préparation comprenant de la gelée royale est dépourvue de conservateur et avantageusement également dépourvue de composé de synthèse. Ceci représente un avantage significatif eu égard aux problèmes - et potentiels problèmes - sanitaires liés à l'emploi de conservateur(s) (cf. supra). Avantageusement, la préparation à base de pulpe de fruit de baobab est également dépourvue de conservateur (et préférablement de composé de synthèse), pour les raisons exposées précédemment.

L'invention a également trait au produit directement obtenu par le procédé thermique de stabilisation (ou de fluidification) et de décontamination susvisé.

L'invention concerne également un procédé thermique de décontamination d'une préparation comprenant de la gelée royale telle que définie précédemment (et en particulier de la gelée royale comprise dans ladite préparation), ledit procédé comprenant les étapes suivantes :

a) obtenir ladite préparation comprenant de la gelée royale, de préférence comprenant, consistant essentiellement en, ou consistant en, un mélange de gelée royale et d'eau, de préférence dans un rapport en masse gelée royale/eau compris entre 5/200 et 1/2, préférablement entre 5/100 et 2/5, avantageusement entre 2/25 et 3/10,

b) introduire ladite préparation au sein d'un récipient, de préférence obturable, avantageusement de manière hermétique, ledit récipient étant adapté pour réaliser un traitement thermique de décontamination tel qu'un traitement de type pasteurisation ou stérilisation, préférablement de type pasteurisation,

c) à l'étape b), ou de préférence avant ladite étape b) (par exemple à l'étape a)), mélanger ladite préparation comprenant de la gelée royale avec ladite au moins une préparation à base de pulpe de fruit de baobab telle que définie précédemment,

d) postérieurement à l'étape b), effectuer, au sein dudit récipient, un traitement thermique de type pasteurisation ou stérilisation, de préférence de type pasteurisation, avantageusement à une température comprise entre environ 60°C et environ 99°C, préférablement à une température comprise entre environ 75°C et environ 95°C.

Selon un mode de réalisation, lorsque ladite préparation comprenant de la gelée royale est une préparation comprenant un mélange de gelée royale et d'eau, ladite au moins une préparation à base de pulpe de fruit de baobab est, de préférence, introduite dans l'eau de ladite préparation comprenant de la gelée royale concomitamment - ou simultanément - à la gelée royale (à l'étape a)).

L'invention a également trait au produit directement obtenu par le procédé thermique de décontamination susvisé.

Selon un mode de réalisation, à l'étape c), le mélange est réalisé dans un rapport en masse préparation à base de pulpe de fruit de baobab/gelée royale d'au moins 5/1000 (par exemple supérieur à 5/1000), de préférence d'au moins 1/100, préférablement d'au moins 1/75, avantageusement d'au moins 3/200 (par exemple supérieur à 3/200), de manière préférée d'au moins 1,8/100, et de manière particulièrement préférée d'au moins 1/50.

Selon un mode de réalisation, le rapport en masse préparation à base de pulpe de fruit de baobab/gelée royale est au maximum de 1/10 (par exemple inférieur à 1/10), de préférence au maximum de 7/100 et avantageusement au maximum de 5/100.

Selon un mode de réalisation, au moins x% de ladite au moins une préparation à base de pulpe de fruit de baobab, défini(s) par rapport à la masse de gelée royale, est/sont mélangé(s), à l'étape c), avec ladite préparation comprenant de la gelée royale pour atteindre une viscosité cinématique critique prédéterminée y, à partir de laquelle l'écoulement du mélange est optimal, ledit/lesdits x% étant déterminé(s) par la relation suivante :

$$x\% = \frac{y + 14,222}{22,248}$$

L'invention a également pour objet un procédé permettant de déterminer, dans une composition comprenant (consistant essentiellement, ou consistant en) au moins une préparation à base de pulpe de fruit de baobab telle que définie précédemment et une préparation comprenant de la gelée royale telle que définie précédemment, le pourcentage en masse (x%) de ladite au moins une préparation à base de pulpe de fruit de baobab, défini par rapport à la masse de gelée royale, permettant d'atteindre une viscosité cinématique critique prédéterminée y, ledit procédé comprenant :

- déterminer, par tout moyen, une viscosité cinématique critique y à atteindre (pour obtenir un écoulement optimal de la composition), et
- déterminer le pourcentage en masse (x%) de ladite au moins une préparation à base de pulpe de fruit de baobab, en appliquant l'équation suivante :

$$x\% = \frac{y + 14,222}{22,248}$$

Selon un mode de réalisation, l'invention concerne un procédé tel que défini précédemment, dans lequel :

- ladite préparation comprenant de la gelée royale comprend, consiste essentiellement en, ou consiste en, 1000 mg de gelée royale et, à l'étape c), le mélange est réalisé dans un rapport en masse préparation à base de pulpe de fruit de baobab/gelée royale d'au moins 5/1000 (par exemple supérieur à 5/1000), de préférence d'au moins 1/100, ou
- ladite préparation comprenant de la gelée royale comprend, consiste essentiellement en, ou consiste en, 1500 mg de gelée royale et, à l'étape c), le mélange est réalisé dans un rapport en masse préparation à base de pulpe de fruit de baobab/gelée royale d'au moins 5/1000 (par exemple supérieur à 5/1000), de préférence d'au moins 1/100, préférablement d'au moins 1/75, ou
- ladite préparation comprenant de la gelée royale comprend, consiste essentiellement en, ou consiste en, 2000 mg de gelée royale et, à l'étape c), le mélange est réalisé dans un rapport en masse préparation à base de pulpe de fruit de baobab/gelée royale d'au moins 1/100 (par exemple supérieur à 1/100), de préférence d'au moins 3/200, préférablement d'au moins 1/50, et avantageusement d'au moins 5/200.

Le procédé thermique selon l'invention présente notamment l'avantage de permettre d'optimiser/de préserver efficacement la qualité microbiologique de la préparation comprenant la gelée royale, tout en :

- préservant la qualité de la gelée royale (en particulier sa qualité nutritive), ses caractéristiques physico-chimiques, et également, de manière avantageuse, ses propriétés organoleptiques,
- évitant (ou limitant drastiquement) le phénomène de gélification se produisant lorsque la préparation comprenant de la gelée royale est soumise à un traitement thermique (par exemple de décontamination), tout en
- étant relativement peu onéreux, facile à mettre en œuvre et en ne nécessitant pas d'appareillage spécifique/complexe.

Définitions

*Gelée Royale.* La gelée royale est une substance blanchâtre et gélatineuse sécrétée par les glandes hypopharyngiennes et mandibulaires des abeilles *(Apis mellifera* L.) ouvrières nourricières, qui sert à la nutrition des larves au premier stade de leur développement et des reines durant toute leur existence [Khazaei et al., 2017 **[1]** ; Fratini et al., 2016 **[2]].** C'est une substance active d'origine naturelle issue de la ruche, bien connue de l'homme du métier.

De préférence, la gelée royale - et sa qualité - est définie au sein de la norme NF ISO 12824 **[3].**

Selon un mode de réalisation, la gelée royale utilisée aux fins de la présente invention présente les caractéristiques physico-chimiques suivantes :

- Eau : 62.0 - 68.5%
- Cendre : $\leq$ 3.0 %
- Acidité totale : 30.0 - 53.0 mmol/100g
- Isotopes C13 et C12 : -29 to -20
- 10-2-HDA (acide 10-hydroxy-2-décénoïque ; n°CAS : 14113-05-4) : $\geq$ 1.4 g/100g

De manière alternative ou complémentaire (avantageusement de manière complémentaire), la gelée royale utilisée aux fins de la présente invention possède les propriétés nutritionnelles suivantes :

- Protéines : 11 - 18 %
- Lipides : 2 - 8 %
- Sucre total : 7 - 18 %
- Glucose : 2 - 9 %
- Fructose : 2 -9 %
- Saccharose : $\leq$ 3.0 %
- Erlose : $\leq$ 0.5 %
- Maltose : $\leq$ 1.5 %
- Maltotriose : $\leq$ 0.5 %

Selon un mode de réalisation, la gelée royale utilisée aux fins de la présente invention présente les caractéristiques organoleptiques suivantes :

- Couleur : blanc perle à jaune
- Apparence : liquide opaque épais
- Goût : sucré légèrement épicé

*Baobab.* Les baobabs (genre *Adansonia,* famille des *Bombacaceae)* sont des arbres tropicaux adaptés à la survie dans des environnements possédant une saison humide très pluvieuse et une saison sèche très aride : ils perdent leurs feuilles en saison sèche et stockent de grandes quantités d'eau dans leur tronc, qui prend souvent une allure de bouteille voire dans certains cas de ballon de baudruche. Le genre comporte plusieurs espèces, par exemple *Adansonia digitata* L., le baobab africain, *Adansonia gregorii* F.Muell, le baobab australien, *Adansonia madagascariensis* Baill., le baobab malgache, etc.

Le baobab africain *(Adansonia digitata* L.) est probablement l'arbre d'Afrique le plus connu et l'un des plus anciens. Il s'agit d'un arbre typique de la savane arborée sèche, dénommé par les populations locales : « l'arbre de vie ». Sa silhouette est distinctive, son tronc large, ses branches ressemblent à des racines et ses gros fruits sont veloutés.

*Pulpe de fruit de baobab (ou « pulpe de fruit du baobab »).* La pulpe de fruit de baobab africain est utilisée en médecine traditionnelle. Sous une coque rigide, le fruit du baobab (également dénommé « pain de singe » ; généralement de forme ovoïde) renferme des graines enveloppées de pulpe blanche. Cette pulpe est utilisée aux fins de la présente invention. Elle présente une valeur nutritive élevée de par sa richesse en calcium, vitamines, minéraux et oligo-éléments (Jackson, 2015 **[4])**. Les principales vitamines présentes dans la pulpe sont la vitamine C et différentes vitamines B, notamment les vitamines B1, B2, B3 et B6 (Diop et al., 2006 **[5])**. Selon la présente invention, la pulpe de fruit de baobab est utilisée sous forme de préparation à base de plante(s) (préparation à base de pulpe de fruit de baobab) et en particulier sous forme de poudre de pulpe de fruit de baobab ou d'extrait de pulpe de fruit de baobab.

Selon un mode de réalisation préféré, l'on utilise, aux fins de la présente invention, la pulpe de fruit du baobab africain *(Adansonia digitata* L.), par exemple sous forme de préparation pulvérulente (poudre).

*Préparation à base de plante(s).* Une préparation à base de plante(s) est une préparation appropriée obtenue à partir d'une substance active d'origine végétale par un traitement comme l'extraction, la distillation, l'expression, le fractionnement, la réduction de la taille, la purification, la concentration ou la fermentation [Ph. Eur., 07/20210:1434] (ou encore par déshydratation (par exemple par lyophilisation)). Les procédés de traitement sont par exemple mécaniques (pressage, filtration, évaporation...), physico-chimiques (extraction par solvant, entraînement à la vapeur, ou distillation) et/ou biochimiques, permettant d'extraire certains constituants phytochimiques d'intérêt. Les préparations à base de plante(s) incluent, par exemple, les extraits, les huiles essentielles, les jus pressés, les exsudats traités, les plantes coupées ou pulvérisées [Ph. Eur., 07/20210:1434]. Dans le cas des fruits, il peut s'agir de concentrés, tel que des concentrés de jus, d'infusions concentrées ou d'alcoolats. Ces concentrés sont obtenus à partir d'une substance active d'origine végétale (fruit) par un procédé d'extraction mécanique d'évaporation d'eau.

*Poudre de pulpe de fruit de baobab.* Tel qu'indiqué précédemment, et selon un mode de réalisation préféré de l'invention, la pulpe de fruit de baobab est utilisée sous forme de poudre de pulpe de fruit de baobab (de préférence se présentant sous forme de particules micronisées), à savoir de poudre obtenue à partir de la pulpe du fruit du baobab, de préférence par séparation mécanique (en particulier par séparation mécanique de la pulpe et des graines) et pulvérisation (avantageusement par micronisation de la pulpe), naturellement déshydratée à l'intérieur du fruit. Selon un mode de réalisation préféré, le procédé de préparation de la susdite poudre de pulpe de fruit de baobab est un procédé exclusivement mécanique. De préférence, aucun conservateur ni composé de synthèse (en particulier aucun solvant organique d'extraction) n'est utilisé pour obtenir la susdite poudre de pulpe de fruit de baobab. En outre, ce procédé de préparation de poudre de pulpe de fruit de baobab est, de préférence, mis en œuvre en l'absence d'eau.

De préférence, la poudre de pulpe de fruit de baobab se présente sous forme de particules de taille micronique telles que des particules micronisées, de préférence lesdites particules de taille micronique ayant une granulométrie inférieure ou égale à 1000 $\mu$m, préférablement inférieure à 1000 $\mu$m. Avantageusement, lesdites particules ont une granulométrie inférieure à 900 $\mu$m, et de manière préféré inférieure à 800 $\mu$m.

Selon un mode de réalisation préféré, ladite poudre de pulpe de fruit de baobab présente les caractéristiques suivantes :

- environ 40% à environ 50% de fibres alimentaires, soit environ 5% à environ 25% de fibres insolubles dans l'eau et environ 15% à environ 45% de fibres solubles dans l'eau, et/ou
- au moins 70%, de préférence au moins 75%, préférablement au moins 80 %, avantageusement environ 85 %, des particules de ladite poudre ont une taille inférieure à 125 $\mu$m et, avantageusement, environ 30 % des particules de ladite poudre ont une taille inférieure à 90 $\mu$m (distribution granulométrique déterminée par la méthode de la Pharmacopée Européenne [2.9.38, Estimation de la distribution granulométrique par tamisage analytique]).

*Particules de taille micronique.* Particules obtenues par une opération mécanique de broyage permettant la réduction d'un solide en petites particules, en l'occurrence l'obtention d'une poudre très fine de l'ordre du micron (granulométrie d'environ 1 à 1000 $\mu$m). De préférence, lesdites particules sont des particules micronisées, à savoir obtenues par une opération de pulvérisation appelée « micronisation », permettant la réduction d'un solide en petites particules en l'occurrence l'obtention d'une poudre très fine de l'ordre du micron (granulométrie d'environ 1 à 1000 $\mu$m).

*Extrait de pulpe de fruit de baobab.* Tel qu'indiqué précédemment, et selon un mode de réalisation de l'invention, la pulpe de fruit de baobab peut être utilisée sous forme d'extrait à base de plante(s) (extrait de pulpe de fruit de baobab). A titre d'extrait de pulpe de fruit de baobab, il est possible de citer notamment les extraits aqueux, les extraits hydroalcooliques ou les extraits hydroglycérinés.

*Extrait à base de plante(s).* Les extraits à base de plante(s) sont des préparations liquides, semi-solides ou solides, obtenues à partir des substances d'origine végétale, en utilisant un solvant approprié [Ph. Eur., 07/2015:0765].

*(Phénomène de) Gélification de la préparation comprenant la gelée royale.* Même si la gelée royale « fraîche » se présente, par essence, sous forme légèrement gélifiée, l'on observe, dans le temps et/ou lorsqu'un traitement thermique (tel qu'un traitement thermique de décontamination de type pasteurisation ou stérilisation) est appliqué à la gelée royale, une gélification très importante (agglomération de celle-ci). Sans être lié à la théorie, cette gélification apparaît liée à un phénomène de polymérisation se produisant au sein de la gelée royale, vraisemblablement dû à sa teneur importante en protéines (généralement entre 10 et 20% et typiquement entre 11 et 18%) et résultant en la formation d'un agrégat protéique.

*Traitement thermique.* (Procédé de) traitement comprenant au moins une étape d'augmentation/d'élévation de la température durant une période de temps déterminée (appropriée pour permettre la bonne réalisation dudit traitement). De préférence, aux fins de la présente invention, le traitement thermique est un traitement thermique de décontamination, tel qu'une pasteurisation ou une stérilisation.

*Stérilisation.* Lorsque la préparation comprenant de la gelée royale est soumise à un traitement thermique de décontamination, ce traitement peut consister, par exemple, en un procédé de stérilisation. Tel qu'indiqué dans l'instruction technique DGAL/SDSSA/2015-364 du 6 octobre 2015 (publiée le 13 octobre 2015) **[6]** (partie I-2), un procédé de « stérilisation » s'entend d'un «traitement thermique à des températures supérieures à 100°C, visant à détruire les enzymes, les micro-organismes dans leurs formes végétatives et sporulées et leurs toxines thermosensibles, assurant la stabilité à température ambiante des produits ».

*Pasteurisation.* Selon un mode de réalisation préféré de l'invention, la préparation comprenant de la gelée royale est soumise à un traitement thermique de décontamination consistant en un procédé de pasteurisation. Tel que défini au sein de l'instruction technique DGAL/SDSSA/2015-364 du 6 octobre 2015 (publiée le 13 octobre 2015) [6] (partie I-2), la pasteurisation est : un « traitement thermique à des températures inférieures à 100°C, visant à détruire les enzymes et les micro-organismes dans leur forme végétative ».

Tel qu'indiqué précédemment, et selon un mode de réalisation particulièrement préféré de l'invention, le traitement thermique de la préparation comprenant de la gelée royale est un procédé de pasteurisation. Ce procédé présente notamment l'avantage d'être efficace pour l'optimisation de la qualité microbiologique tout en :

- préservant la qualité de la gelée royale (en particulier sa qualité nutritive), ses caractéristiques physico-chimiques, et également, de manière avantageuse, ses propriétés organoleptiques, et en
- étant relativement peu onéreux.

*Conservateur.* Par conservateur, l'on entend, au sens de la présente invention, un ingrédient chimique capable d'empêcher l'altération chimique et/ou d'inhiber le développement de microorganismes.

*Composé de synthèse (ou composé d'origine synthétique).* Composé issu de la chimie de synthèse (non naturel ou d'origine non naturelle). Egalement dénommé communément « ingrédient chimique ».

*Dispositif médical:* Tout instrument, appareil, équipement, logiciel, implant, réactif, matière ou autre article, destiné par le fabricant à être utilisé, seul ou en association, chez l'homme pour l'une ou plusieurs des fins médicales précises suivantes: - diagnostic, prévention, contrôle, prédiction, pronostic, traitement ou atténuation d'une maladie, - diagnostic, contrôle, traitement, atténuation d'une blessure ou d'un handicap ou compensation de ceux-ci, - investigation, remplacement ou modification d'une structure ou fonction anatomique ou d'un processus ou état physiologique ou pathologique, - communication d'informations au moyen d'un examen in vitro d'échantillons provenant du corps humain, y compris les dons d'organes, de sang et de tissus, et dont l'action principale voulue dans ou sur le corps humain n'est pas obtenue par des moyens pharmacologiques ou immunologiques ni par métabolisme, mais dont la fonction peut être assistée par de tels moyens (cf. Règlement (UE) 2017/745).

La présente invention trouve à s'appliquer au domaine des dispositifs médicaux.

*Complément alimentaire.* Composition à usage humain ou animal (de préférence à usage humain) dont le but est de compléter le régime normal et qui constitue une source concentrée de nutriments ou d'autres substances ayant un effet nutritionnel ou physiologique, seuls ou combinés, commercialisés sous forme de doses, à savoir les formes de présentation telles que les gélules, les pastilles, les comprimés, les pilules et autres formes similaires, ainsi que les sachets de poudre, les ampoules de liquide, les flacons munis d'un compte-gouttes et les autres formes analogues de préparations liquides ou en poudre destinées à être prises en unités mesurées de faible quantité.

La présente invention trouve à s'appliquer au domaine des compléments alimentaires à usage humain ou animal, de préférence à usage humain.

*Aliments diététiques destinés à des fins médicales spéciales (ADDFMS).* Conformément à la législation française, et plus particulièrement à l'Article L5137-1 du Code de la santé publique, « On entend par aliments diététiques destinés à des fins médicales spéciales les aliments destinés à une alimentation particulière qui sont spécialement traités ou formulés pour répondre aux besoins nutritionnels des patients. Ils sont destinés à constituer l'alimentation exclusive ou partielle des patients dont les capacités d'absorption, de digestion, d'assimilation, de métabolisation ou d'excrétion des aliments ordinaires ou de certains de leurs ingrédients ou métabolites sont diminuées, limitées ou perturbées, ou dont l'état de santé appelle d'autres besoins nutritionnels particuliers qui ne peuvent être satisfaits par une modification du régime alimentaire normal ou par un régime constitué d'aliments destinés à une alimentation particulière ou par une combinaison des deux ».

La présente invention trouve à s'appliquer au domaine des ADDFMS.

*Viscosité (cps ou mPa.s ; également dénommée « viscosité dynamique »).* La viscosité est la mesure de la résistance à l'écoulement d'un fluide. La viscosité est déterminée à l'aide d'un viscosimètre, lequel mesure la viscosité de fluides à des vitesses de cisaillement déterminées. Le principe général de fonctionnement d'un viscosimètre consiste à faire tourner un mobile (immergé dans le fluide dont on souhaite mesurer la viscosité) par l'intermédiaire d'un ressort calibré. La résistance exercée par le fluide contre le mobile est évaluée grâce à la torsion du ressort. Cette dernière est mesurée à l'aide d'un transducteur rotatif. La plage de mesure d'un viscosimètre (en centipoises ou milliPascal•seconde) est déterminée par la vitesse de rotation du mobile, la taille et la forme dudit mobile, le récipient contenant le fluide et la pleine échelle du couple de torsion du ressort calibré.

*Viscosité cinématique (mm$^2$/s).* La viscosité cinématique s'entend du quotient de la viscosité dynamique par la masse volumique du fluide. Cette viscosité cinématique représente la capacité de rétention des particules du fluide et quantifie sa capacité à s'épancher. Elle s'exprime en unité de surface par unité de temps (par exemple en mm$^2$/s).

*Viscosité cinématique critique.* Viscosité cinématique à partir de laquelle l'écoulement d'une substance, d'une composition ou d'un mélange est optimal. Cette viscosité cinématique critique peut être déterminée au moyen de tests de routine en fonction du volume et de la nature de la substance, composition ou mélange considéré(e). Tel qu'indiqué précédemment, les inventeurs ont découvert que, pour une composition comprenant un mélange entre une préparation à base de pulpe de fruit de baobab et une préparation comprenant de la gelée royale, le pourcentage en masse de préparation à base de pulpe de fruit de baobab (x%) (défini par rapport à la masse de la gelée royale) et la viscosité cinématique critique y de la composition dans son ensemble sont liés par la relation suivante :

$$x\% = \frac{y + 14{,}222}{22{,}248}$$

**Brève description des dessins**

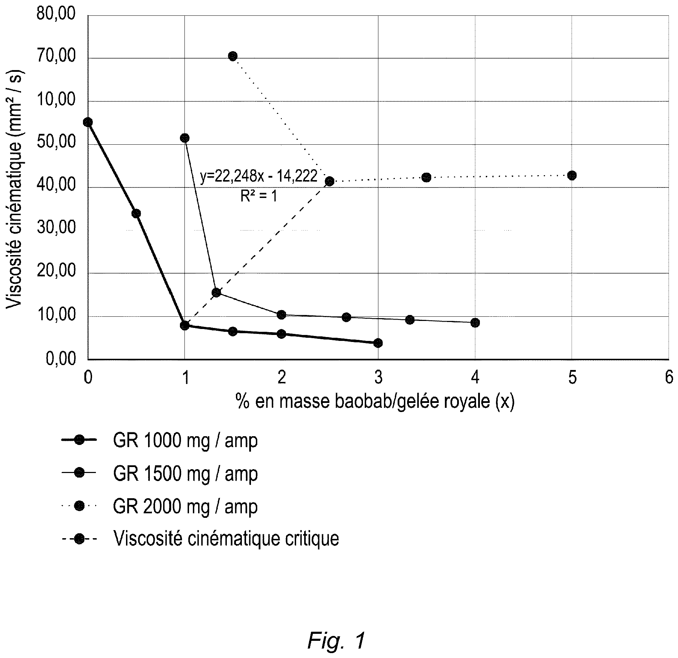

L'invention, sa fonctionnalité, ses applications ainsi que ses avantages seront mieux appréhendés à la lecture de la présente description, faite en référence à la figure 1 correspondant à la représentation graphique discutée au sein de l'exemple 4 infra et établissant notamment la relation entre viscosité cinématique critique (mm$^2$/s) et pourcentage en masse de poudre de pulpe de fruit de baobab par rapport à la masse de gelée royale utilisée.

**Description détaillée**

Les exemples ci-après permettront de mieux appréhender la présente invention.

**Exemples**

Exemple 1 : Tests visant à déterminer d'éventuelles variations concernant le phénomène de gélification observé lors du traitement thermique de la gelée royale

Différents essais ont été réalisés, les paramètres suivants ont été testés :

- Matière : interactions matières, gelée royale non bio et lyophilisée, concentration,
- Matériel : conditionnement ampoule / UNICADOSE®,
- Milieu : eau de ville / eau purifiée, acidité, viscosité, et
- Méthode : pré-mélange, température de mélange, traitement thermique.

Les tests ont tous été réalisées sous la référence MELANGE GELEE ROYALE BIO contenant 1500 mg de gelée royale par ampoule et dont la composition est mentionnée dans le tableau 6 infra.

Tableau 6

| Désignation | % en masse |
|---|---|
| EAU DE VILLE | 72.910 |
| MIEL DE FLEUR BIO /CAT7 | 5.000 |
| JUS D'ORANGE CONCENTRE BIO | 4.000 |
| SIROP BLE BIO TYPE B 17,5K | 9.000 |
| GELEE ROYALE BIO + | 9.090 |

1.1. Eventuelle influence de la nature de la gelée royale sur le phénomène de gélification

L'on utilise une formulation à 1500 mg de gelée royale (GR) par ampoule de 10 ml (ampoule en verre à extrémités cassables). Une gélification importante de la gelée royale dans l'ampoule est observée après traitement thermique par pasteurisation.

L'on met en œuvre le mode opératoire suivant :

- la quantité d'eau froide nécessaire est introduite dans un mélangeur sous agitation ;
- sous agitation, les constituants du mélange, à savoir la gelée royale, le miel bio, le sirop de blé bio, le jus d'orange bio, sont solubilisés dans l'eau afin d'obtenir un mélange homogène ;
- les paramètres physico-chimiques de ce mélange sont la couleur orange, le pH qui est compris entre 3,5-4,0 ; et une densité de 1,1 ; ces paramètres justifient l'homogénéité du mélange ;
- la répartition du mélange est effectuée en ampoule de verre de 10 ml à extrémités cassables, tel qu'indiqué précédemment ; puis
- un traitement thermique par pasteurisation (à environ 90°C durant environ 60 minutes) est ensuite réalisé afin d'optimiser la qualité et de protéger le mélange d'un point de vue microbiologique.

Au sein de la composition présentée dans le tableau 6 supra, la gelée royale bio a été substituée par les gelées royales suivantes :

- gelée royale (non bio),
- gelée royale lyophilisée,
- gelée royale, origine France, et
- gelée royale provenant de trois fournisseurs différents.

L'étude a révélé que le phénomène de gélification dû au procédé de traitement thermique (de décontamination) se produisait quelles que soient la nature et la source de la gelée royale. L'invention trouve donc à s'appliquer à tous les types et à toutes les sources de gelée royale.

1.2 Influence éventuelle du contenant

L'on met en œuvre le mode opératoire suivant :

- la quantité d'eau froide nécessaire est introduite dans un mélangeur sous agitation ;
- sous agitation, les constituants du mélange, à savoir la gelée royale, le miel bio, le sirop de blé bio, le jus d'orange bio, sont solubilisés dans l'eau afin d'obtenir un mélange homogène ;
- les paramètres physico-chimiques de ce mélange sont la couleur orange, le pH qui est compris entre 3,5-4,0; et une densité de 1,1 ; ces paramètres justifient l'homogénéité du mélange ;
- la répartition du mélange est effectuée en UNICADOSE® en propylène de 15 ml ; puis
- un traitement thermique par pasteurisation (à environ 90°C durant environ 60 minutes) est ensuite réalisé afin d'optimiser la qualité et de protéger le mélange d'un point de vue microbiologique.

La composition présentée dans le tableau 6 supra a été conditionnée 1) en ampoule en verre à extrémités cassables et en UNICADOSES®.

Il a été constaté un phénomène de gélification important quel que soit le type de conditionnement. Celui-ci n'apparaît donc pas influer sur le phénomène de gélification.

1.3. Eventuelle influence du milieu sur le phénomène de gélification

L'on met en œuvre le mode opératoire suivant :

- la quantité d'eau froide nécessaire est introduite dans un mélangeur sous agitation ;
- sous agitation, les constituants du mélange, à savoir la gelée royale, le miel bio, le sirop de blé bio, le jus d'orange bio, sont solubilisés dans l'eau afin d'obtenir un mélange homogène ;
- les paramètres physico-chimiques de ce mélange sont la couleur orange, le pH qui est compris entre 3,5-4,0; et une densité de 1,1 ; ces paramètres justifient l'homogénéité du mélange ;
- la répartition du mélange est effectuée en ampoule de verre de 10 ml à extrémités cassables, tel qu'indiqué précédemment ; puis

- un traitement thermique par pasteurisation (à environ 90°C durant environ 60 minutes) est ensuite réalisé afin d'optimiser la qualité et de protéger le mélange d'un point de vue microbiologique.

Une étude a également été effectuée afin de tenter de déterminer l'influence potentielle de la qualité de l'eau utilisée dans la composition présentée au sein du tableau 6 supra. L'eau de ville a été comparée à de l'eau purifiée.

L'influence du pH a également été analysée, en faisant varier le pH de la composition présentée au sein du tableau 6 supra. Divers excipients ont également été ajoutés afin de tenter de fluidiser ladite composition.

En conclusion de cette étude, il a été constaté, dans tous les cas, un phénomène de fluidification important au sein des ampoules ; la qualité de l'eau utilisée, les variations de pH et les divers excipients testés n'apparaissant pas avoir d'influence sur le phénomène de gélification.

1.4. Eventuelle influence du procédé sur le phénomène de gélification

L'on met en œuvre le mode opératoire suivant :

- la quantité d'eau froide nécessaire est introduite dans un mélangeur sous agitation ;
- sous agitation, les constituants du mélange, à savoir la gelée royale, le miel bio, le sirop de blé bio, le jus d'orange bio, sont solubilisés dans l'eau afin d'obtenir un mélange homogène ;
- les paramètres physico-chimiques de ce mélange sont la couleur orange, le pH qui est compris entre 3,5-4,0; et une densité de 1,1 ; ces paramètres justifient l'homogénéité du mélange ;
- la répartition du mélange est effectuée en ampoule de verre de 10 ml à extrémités cassables, tel qu'indiqué précédemment ; puis
- un traitement thermique par pasteurisation et/ou stérilisation est ensuite réalisé afin d'optimiser la qualité et de protéger le mélange d'un point de vue microbiologique.

Une étude a également été effectuée afin de tenter de déterminer l'éventuelle influence du procédé de traitement thermique sur le phénomène de gélification. Différents programmes de traitement thermique ont été testés sur la composition objet du tableau 6 supra.

En particulier, les traitements thermiques de décontamination suivants ont été testés :

- pasteurisation à environ 90°C pendant environ 60 minutes,
- stérilisation à environ 104°C pendant environ 60 minutes, et
- stérilisation à environ 120°C pendant environ 25 minutes.

L'influence de la température de fabrication de la composition objet du tableau 6 a également été évaluée, ladite composition ayant été testée en faisant varier les températures de 25°C à 60°C et de 25°C à 80°C.

Par ailleurs, le protocole de fabrication de la composition objet du tableau 6 a été étudié afin de définir l'ordre d'incorporation des matières, ainsi que la possibilité d'utiliser des pré-mélanges.

Les résultats obtenus ont montré, dans tous les cas, une gélification importante même s'il apparaît que la température pourrait avoir une influence sur la texture du produit.

1.5. Eventuelle influence du matériel sur le phénomène de gélification

L'on met en œuvre le mode opératoire suivant :

- la quantité d'eau froide nécessaire est introduite dans un mélangeur sous agitation ;
- sous agitation, les constituants du mélange, à savoir la gelée royale, le miel bio, le sirop de blé bio, le jus d'orange bio, sont solubilisés dans l'eau afin d'obtenir un mélange homogène ;
- les paramètres physico-chimiques de ce mélange sont la couleur orange, le pH qui est compris entre 3,5-4,0; et une densité de 1,1 ; ces paramètres justifient l'homogénéité du mélange ;
- la répartition du mélange est effectuée en ampoule de verre de 10 ml à extrémités cassables, tel qu'indiqué précédemment ; puis
- un traitement thermique par pasteurisation (à environ 90°C durant environ 60 minutes) est ensuite réalisé afin d'optimiser la qualité et de protéger le mélange d'un point de vue microbiologique.

En dernier lieu, une étude a été également été réalisée pour déterminer l'influence du matériel, et en particulier des mélangeurs utilisés sur le susdit phénomène de gélification. Les techniques d'agitation suivantes ont été testées : agitateurs à hélices (Ystral), à turbine (Ultra Turax ou Sylverson), ultrasons. Aucune d'entre elles ne permet de supprimer - voire même de limiter - la gélification après traitement thermique.

Les résultats de cette étude ont montré qu'un phénomène de gélification important se produisait dans les ampoules quel que soit le mélangeur utilisé, même si de légères différences pouvaient exister.

Exemple 2 : Mise en évidence de l'effet anti-gélifiant de la poudre de pulpe de fruit de baobab sur la gelée royale soumise à un traitement thermique

A titre liminaire, il convient de noter que la poudre de pulpe de fruit de baobab utilisée aux fins du présent exemple et des exemple suivants est d'origine africaine (à savoir obtenue à partir du fruit du baobab africain, *Adansonia digitata L.)* et, plus précisément, présente les caractéristiques suivantes : elle contient 40 à 50% de fibres alimentaires soit environ 5 à 25% de fibres insolubles dans l'eau et 15 à 45% de fibres solubles dans l'eau, et présente la distribution granulométrique suivante, déterminée par la méthode de la Pharmacopée Européenne (2.9.38, Estimation de la distribution granulométrique par tamisage analytique) : environ 85% des particules avec une taille inférieure à 125 $\mu$m et environ 30% des particules avec une taille inférieure à 90 $\mu$m.

2.1 Objectif

Afin de mettre en évidence l'effet anti-gélifiant de la poudre de pulpe de fruit de baobab gélifiant (abrégé ci-après en « baobab » ou « poudre de baobab », à des fins de concision) sur la gelée royale soumise à un traitement thermique, des mesures d'écoulement ont été effectuées à l'aide d'une coupe d'écoulement normée en essayant de trouver la coupe qui permette d'être dans le domaine de mesure défini (entre 30 et 100s) pour un maximum de formules couvrant les trois concentrations testées de gelée royale (1000, 1500 et 2000 mg/10 ml). La coupe d'écoulement qui remplit cet objectif est la coupe n°3 (3 mm, cf. NF EN ISO 2431, 5.1.4 et 5.1.5 **[7]).**

2.2 Mode opératoire

Agiter vigoureusement dans un bécher :

i) soit 2000 mg de gelée royale + eau qsp 10 ml,
ii) soit 2000 mg de gelée royale + 0,1% en masse de poudre de baobab + eau qsp 10 ml.

Chauffer à 60°C pendant 30 minutes chacune des deux compositions i) et ii) supra. Vider ensuite l'échantillon thermostaté dans la coupelle Elcometer ISO 3 (soit environ 100 ml) en bouchant l'ouverture avec le doigt. Remplir à ras bord et faire le niveau à l'aide d'une lame en verre (NF EN ISO 2431, 7.2.4 **[7]).** Mesurer le temps d'écoulement de l'échantillon (NF EN ISO 2431, 7.2.5 **[7]).**

Remarque : Même si la norme NF EN ISO 2431 **[7]** concerne principalement la détermination du temps d'écoulement au moyen de coupes d'écoulement de peintures et vernis, les inventeurs ont découvert que les modes opératoires et appareillages mentionnés au sein de cette norme étaient particulièrement bien adaptés à la détermination du temps d'écoulement de préparations comprenant de la gelée royale.

2.3 Résultats

Les résultats sont présentés dans le tableau 7 infra :

Tableau 7

| Essai : Méthode de préparation | Désignation | Proportion baobab (%) | Durée écoulement (s) | Viscosité cinématique (mm$^2$/s) |
|---|---|---|---|---|
| Chauffage 30 min à 60°C | GR 2000 mg | 0 | 103 | 43.69 |
| | GR 2000 mg + baobab | 0,1 | 47 | 16.57 |

Dès que l'on épaissit la préparation par chauffage, l'on voit instantanément l'influence de la poudre de pulpe de fruit de baobab dans la composition ; le temps d'écoulement est diminué de moitié.

Exemple 3 : Relation entre quantité de poudre de pulpe de fruit de baobab ajoutée, viscosité de la composition et écoulement de celle-ci

3.1 Principe

La viscosité a été déterminée à l'aide d'un viscosimètre DV2T. Le principe de fonctionnement du viscosimètre DV2T consiste à faire tourner un mobile (immergé dans un fluide) par l'intermédiaire d'un ressort calibré. La résistance exercée par le fluide contre le mobile est évaluée grâce à la torsion du ressort. Cette dernière est mesurée à l'aide d'un transducteur rotatif. La plage de mesure d'unDV2T (en centipoises ou milliPascal•seconde) est déterminée par la vitesse de rotation du mobile, la taille et la forme du mobile, le récipient contenant le fluide et la pleine échelle du couple de torsion du ressort.
Toutes les mesures de viscosité sont réalisées avec le mobile S62 entre 2.5 et 5 rpm afin de comparer les mesures entre elles.

3.2 Mode opératoire

Les différentes compositions testées au sein de cet exemple (cf. tableau 8 infra) sont obtenues en mettant en œuvre le mode opératoire suivant :

- la quantité d'eau froide nécessaire est introduite dans un mélangeur sous agitation, et
- sous agitation, les constituants du mélange, à savoir la gelée royale et la poudre de pulpe de fruit de baobab (lorsque celle-ci est présente au sein de la composition testée) sont solubilisés dans l'eau afin d'obtenir un mélange homogène.

Une gamme de concentration en gelée royale dans des ampoules en verre de 10 ml (à extrémités cassables) a été effectuée. Les concentrations suivantes en gelée royale ont été testées : 1000 mg/10 ml, 1500 mg/10 ml et 2000 mg/10 ml.
Pour chaque concentration en gelée royale (1000 mg/10 ml, 1500 mg/10 ml et 2000 mg/10 ml), une gamme de concentration en poudre de pulpe de fruit de de baobab (abrégée ci-après en « baobab » ou « poudre de baobab », dans un souci de concision) a été effectuée. Les viscosités (en cps) ont été mesurées à l'aide du viscosimètre DV2T susvisé et les observations concernant l'écoulement de la composition en sortie d'ampoule consignées.
Dans un souci d'exhaustivité les lots testés sont présentés dans le tableau 8 infra :

Tableau 8

| N° de lot | Désignation |
|---|---|
| 19 23 05 1/CSQU | Ampoule Gelée royale 1000 mg (sans poudre baobab) |
| 19 06 05 1/CSQU | Ampoule Gelée royale 1000 mg + 5 mg poudre baobab |
| 19 06 05 2/CSQU | Ampoule Gelée royale 1000 mg + 10 mg poudre baobab |
| 19 06 05 3/CSQU | Ampoule Gelée royale 1000 mg + 15 mg poudre baobab |
| 19 06 05 4/CSQU | Ampoule Gelée royale 1000 mg + 20 mg poudre baobab |
| 19 06 05 5/CSQU | Ampoule Gelée royale 1000 mg + 30 mg poudre baobab |
| 19 23 05 3/CSQU | Ampoule Gelée royale 1500 mg (sans poudre baobab) |
| 19 23 05 4/CSQU | Ampoule Gelée royale 1500 mg + 15 mg poudre baobab |
| 19 23 05 5/CSQU | Ampoule Gelée royale 1500 mg + 20 mg poudre baobab |
| 19 23 05 6/CSQU | Ampoule Gelée royale 1500 mg + 30 mg poudre baobab |
| 19 23 05 7/CSQU | Ampoule Gelée royale 1500 mg + 40 mg poudre baobab |
| 19 23 05 8/CSQU | Ampoule Gelée royale 1500 mg + 50 mg poudre baobab |
| 19 23 05 9/CSQU | Ampoule Gelée royale 1500 mg + 60 mg poudre baobab |
| 19 23 05 2/CSQU | Ampoule Gelée royale 2000 mg (sans poudre baobab) |
| 19 07 05 1/CSQU | Ampoule Gelée royale 2000 mg + 10 mg poudre baobab |
| 19 07 05 2/CSQU | Ampoule Gelée royale 2000 mg + 20 mg poudre baobab |

(suite)

| N° de lot | Désignation |
|---|---|
| 19 07 05 3/CSQU | Ampoule Gelée royale 2000 mg + 30 mg poudre baobab |
| 19 07 05 4/CSQU | Ampoule Gelée royale 2000 mg + 50 mg poudre baobab |
| 19 07 05 5/CSQU | Ampoule Gelée royale 2000 mg + 70 mg poudre baobab |
| 19 07 05 6 / CSQU | Ampoule Gelée royale 2000 mg + 100 mg poudre baobab |

Les mesures de viscosité sont effectuées avant et après pasteurisation, réalisée à une température d'environ 90°C durant 60 minutes environ.

3.3 Résultats

3.3.1 Concernant la gelée royale à 1000 mg/10 ml

Les résultats concernant la gelée royale à 1000 mg/10 ml sont présentés dans le tableau 9 infra et les résultats concernant les caractéristiques organoleptiques associées aux différentes concentrations en poudre de pulpe de fruit de de baobab sont présentés dans le tableau 10 infra.

Tableau 9

| nom de l'essai | GR 1 g/10 ml | | | | viscosité (à 10 rpm S62 en cps) | | |
|---|---|---|---|---|---|---|---|
| | concentration baobab | GR % | BAOBAB % | EAU % | avant pasteurisation | après pasteurisation | remarques viscosité |
| 19 23 05 1/CSQU | 0 | 9,804 | | 90,196 | 6 | 1356 | écoulement moyen |
| 19 06 05 1/CSQU | 5 mg | 9,804 | 0,049 | 90,147 | 15 | 815,8 | bon écoulement |
| 19 06 05 2/CSQU | 10 mg | 9,804 | 0,098 | 90,098 | 13 | 480 | très bon écoulement |
| 19 06 05 3/CSQU | 15 mg | 9,804 | 0,147 | 90,049 | 15 | 222 | |
| 19 06 05 4/CSQU | 20 mg | 9,804 | 0,196 | 90,000 | 15 | 171 | |
| 19 06 05 5/CSQU | 30 mg | 9,804 | 0,294 | 89,902 | 9 | 123 | |

Tableau 10

| | Caractères physico-chimiques | | |
|---|---|---|---|
| nom de l'essai | Brix | pH | Densité |
| 19 23 05 1/CSQU | 3° | 4,12 | 1,009 |
| 19 06 05 1/CSQU | 3° | 4,10 | 1,007 |
| 19 06 05 2/CSQU | 3° | 4,10 | 1,010 |
| 19 06 05 3/CSQU | 3° | 4,08 | 1,010 |
| 19 06 05 4/CSQU | 3° | 4,06 | 1,011 |
| 19 06 05 5/CSQU | 3° | 4,02 | 1,011 |

A partir de 5 mg de baobab par ampoule, l'on observe une efficacité due au baobab (début d'écoulement), le mélange n'est pas gélifié et le liquide peut sortir de l'ampoule sans difficulté. A partir de 10 mg de baobab (1% en masse par rapport à la masse de la gelée royale), l'on observe une efficacité accrue du baobab en tant qu'anti-gélifiant.

En outre, et conformément aux données présentées dans le tableau 10 supra, ni l'ajout de poudre de pulpe de fruit de de baobab au sein de la composition testée, ni l'augmentation de la concentration en poudre de pulpe de fruit de de baobab au sein de ladite composition n'affectent les paramètres physico-chimiques principaux de manière significative ; ceux-ci demeurent inchangés.

Remarques :

- la composition ne contenant pas de baobab a une viscosité très élevée après pasteurisation,
- la viscosité des mélanges avant pasteurisation est négligeable en comparaison avec celle mesurée après pasteurisation.

3.3.2 Concernant la gelée royale à 1500 mg/10 ml

Les résultats concernant la gelée royale à 1500 mg/10 ml sont présentés dans le tableau 11 infra et les résultats concernant les caractéristiques organoleptiques associées aux différentes concentrations en poudre de pulpe de fruit de de baobab sont présentés dans le tableau 12 infra.

Tableau 11

| nom de l'essai | GR 1,5 g/10 ml | | | | viscosité (à 5 rpm S62 en cps) | | |
|---|---|---|---|---|---|---|---|
| | concentration baobab | GR % | BAOBAB % | EAU % | avant pasteurisation | après pasteurisation | remarques viscosité |
| 19 23 05 3/CSQU | 0 | 14,706 | | 85,294 | 12 | 12657 | très mauvais écoulement |
| 19 23 05 4/CSQU | 15 mg | 14,706 | 0,147 | 85,147 | 15 | 1380 | assez bon écoulement |
| 19 23 05 5/CSQU | 20 mg | 14,706 | 0,196 | 85,098 | 18 | 780 | très bon écoulement |
| 19 23 05 6/CSQU | 30 mg | 14,706 | 0,294 | 85,000 | 27 | 504 | |
| 19 23 05 7/CSQU | 40 mg | 14,706 | 0,392 | 84,902 | 23 | 492 | |
| 19 23 05 8/CSQU | 50 mg | 14,706 | 0,490 | 84,804 | 18 | 420 | |
| 19 23 05 9/CSQU | 60 mg | 14,706 | 0,588 | 84,706 | 24 | 410 | |

Tableau 12

| nom de l'essai | Caractères physico-chimiques | | |
|---|---|---|---|
| | Brix | pH | Densité |
| 19 23 05 3/CSQU | 4° | 4,09 | 1,015 |
| 19 23 05 4/CSQU | 4° | 4,05 | 1,016 |
| 19 23 05 5/CSQU | 4° | 4,03 | 1,016 |
| 19 23 05 6/CSQU | 4° | 4,01 | 1,016 |
| 19 23 05 7/CSQU | 4° | 3,98 | 1,017 |
| 19 23 05 8/CSQU | 4° | 3,97 | 1,017 |
| 19 23 05 9/CSQU | 4° | 3,96 | 1,018 |

A partir de 15 mg de baobab, l'on observe une efficacité due au baobab, le mélange n'est pas gélifié et le liquide peut sortir de l'ampoule sans difficulté.

A partir de 20 mg de baobab, l'on observe une efficacité accrue du baobab en tant qu'anti-gélifiant, le mélange n'est pas gélifié et le liquide peut sortir de l'ampoule sans difficulté.

En outre, et conformément aux données présentées dans le tableau 12 supra, ni l'ajout de poudre de pulpe de fruit de de baobab au sein de la composition testée, ni l'augmentation de la concentration en poudre de pulpe de fruit de de baobab au sein de ladite composition n'affectent les paramètres physico-chimiques principaux de manière significative ; ceux-ci demeurent inchangés.

Remarques :

- la composition ne contenant pas de baobab a une viscosité très élevée après pasteurisation,

- la viscosité des mélanges avant pasteurisation est négligeable en comparaison avec celle mesurée après pasteurisation.

3.3.3 Concernant la gelée royale à 2000 mg/10 ml

Les résultats concernant la gelée royale à 2000 mg/10 ml sont présentés dans le tableau 13 infra et les résultats concernant les caractéristiques organoleptiques associées aux différentes concentrations en poudre de pulpe de fruit de de baobab sont présentés dans le tableau 14 infra.

Tableau 13

| nom de l'essai | GR 2,0 g /10 ml | | | | viscosité avec S62 en cps | | |
|---|---|---|---|---|---|---|---|
| | concentration baobab | GR % | BAOBAB % | EAU % | avant pasteurisation | après pasteurisation | remarques viscosité |
| 19 23 05 2/CSQU | 0 | 19,608 | | 80,392 | 21 | 17515 | aucun écoulement |
| 19 07 05 1/CSQU | 10 mg | 19,608 | 0,098 | 80,294 | 57 | 8200 | mauvais écoulement |
| 19 07 05 2/CSQU | 20 mg | 19,608 | 0,196 | 80,196 | 60 | 3700 | Ecoulement moyen |
| 19 07 05 3/CSQU | 30 mg | 19,608 | 0,294 | 80,098 | 60 | 2180 | assez bon écoulement 5 rpm S62 |
| 19 07 05 4/CSQU | 50 mg | 19,608 | 0,490 | 79,902 | 48 | 1764 | bon écoulement 5 rpm |
| 19 07 05 5/CSQU | 70 mg | 19,608 | 0,686 | 79,706 | 60 | 1290 | bon écoulement 5 rpm |
| 19 07 05 6/CSQU | 100 mg | 19,608 | 0,980 | 79,412 | 72 | 800 | bon écoulement 5 rpm |

Tableau 14

| nom de l'essai | Caractères physico-chimiques | | |
|---|---|---|---|
| | Brix | pH | Densité |
| 19 23 05 2/CSQU | 6° | 4,08 | 1,021 |
| 19 07 05 1/CSQU | 6° | 4,06 | 1,021 |
| 19 07 05 2/CSQU | 6° | 4,04 | 1,023 |

(suite)

| nom de l'essai | Caractères physico-chimiques | | |
| --- | --- | --- | --- |
| | Brix | pH | Densité |
| 19 07 05 3/CSQU | 6° | 4,02 | 1,022 |
| 19 07 05 4/CSQU | 6° | 3,96 | 1,024 |
| 19 07 05 5/CSQU | 6° | 3,94 | 1,025 |
| 19 07 05 6/CSQU | 6° | 3,90 | 1,026 |

A partir de 20 mg de baobab, l'on observe une efficacité due au baobab, le mélange n'est pas gélifié et le liquide peut sortir de l'ampoule sans difficulté.

A partir de 30 mg de baobab, l'on observe une efficacité accrue du baobab en tant qu'anti-gélifiant, le mélange n'est pas gélifié et le liquide peut sortir de l'ampoule sans difficulté.

En outre, et conformément aux données présentées dans le tableau 14 supra, ni l'ajout de poudre de pulpe de fruit de de baobab au sein de la composition testée, ni l'augmentation de la concentration en poudre de pulpe de fruit de de baobab au sein de ladite composition n'affectent les paramètres physico-chimiques principaux de manière significative ; ceux-ci demeurent inchangés.

Remarques :

- la formulation ne contenant pas de baobab a une viscosité très élevée après pasteurisation,
- la viscosité des mélanges avant pasteurisation est négligeable en comparaison avec celle mesurée après pasteurisation.

Exemple 4 : Mesures d'écoulement

4.1 Objectif

Afin de mieux comprendre et caractériser le comportement de la poudre de pulpe de fruit de baobab en tant qu'anti-gélifiant (désigné ci-après « baobab » ou « poudre de baobab »), des mesures d'écoulement ont été effectuées.

L'objectif est ici de mesurer l'écoulement des différentes formules présentées dans le tableau 8 supra à l'aide d'une coupe d'écoulement normée en essayant de trouver la coupe qui permette d'être dans le domaine de mesure défini (entre 30 et 100s) pour un maximum de formules couvrant les 3 concentrations testées de gelée royale (1000, 1500 et 2000 mg / 10ml). La coupe d'écoulement qui remplit cet objectif est la coupe n°3 (3 mm, cf. NF EN ISO 2431 **[7]**, 5.1.4 et 5.1.5)

4.2 Mode opératoire

Agiter vigoureusement les ampoules préalablement pasteurisées. Vider le contenu de 12 ampoules dans un bécher afin d'obtenir a minima 100 ml d'échantillon. Thermostater l'échantillon dans un bain thermostaté à 25°C au minimum 30 minutes. Puis vider l'échantillon thermostaté dans la coupelle Elcometer ISO 3 (soit environ 100 ml) en bouchant l'ouverture avec le doigt. Remplir à ras bord et faire le niveau à l'aide d'une lame en verre (NF EN ISO 2431 **[7]**, 7.2.4). Mesurer le temps d'écoulement de l'échantillon (NF EN ISO 2431 [7], 7.2.5).

4.3 Résultats

Les résultats obtenus sont présentés dans le tableau 15 infra :
Etude réalisée sur coupelle Elcometer ISO 3 SE17087 à 25°C
Référentiel NF EN ISO 2431 **[7]**
Plage d'utilisation de la coupelle : $30 \leq t(s) \leq 100$

Tableau 15

| Essai | Désignation | Proportion baobab (%) | Durée écoulement (s) | Viscosité cinématique (mm$^2$/s) |
|---|---|---|---|---|
| 192305 1/CSQU | GR 1000 mg / amp | 0 | 128 | 55.14 |
| 190605 1/CSQU | GR 1000 mg / amp + 5 mg baobab | 0.5 | 82 | 33.89 |
| 190605 2/CSQU | GR 1000 mg / amp + 10 mg baobab | 1 | 32 | 7.93 |
| 190605 3/CSQU | GR 1000 mg / amp + 15 mg baobab | 1.5 | 30 | 6.62 |
| 190605 4/CSQU | GR 1000 mg / amp + 20 mg baobab | 2 | 29 | 5.95 |
| 190605 5/CSQU | GR 1000 mg / amp + 30 mg baobab | 3 | 26 | 3.83 |
| 192305 3/CSQU | GR 1500 mg / amp | 0 | 2040 | 903.62 |
| 192305 4/CSQU | GR 1500 mg / amp + 15 mg baobab | 1 | 120 | 51.49 |
| 192305 5/CSQU | GR 1500 mg / amp + 20 mg baobab | 1.33 | 45 | 15.49 |
| 192305 6/CSQU | GR 1500 mg / amp + 30 mg baobab | 2 | 36 | 10.39 |
| 192305 7/CSQU | GR 1500 mg / amp + 40 mg baobab | 2.67 | 35 | 9.79 |
| 192305 8/CSQU | GR 1500 mg / amp + 50 mg baobab | 3.33 | 34 | 9.18 |
| 192305 9/CSQU | GR 1500 mg / amp + 60 mg baobab | 4 | 33 | 8.56 |
| 192305 2/CSQU | GR 2000 mg / amp | 0 | NA | #VALEUR! |
| 190705 1/CSQU | GR 2000 mg / amp + 10 mg baobab | 0.5 | NA | #VALEUR! |
| 190705 2/CSQU | GR 2000 mg / amp + 20 mg baobab | 1 | 436 | 192.69 |
| 190705 3/CSQU | GR 2000 mg / amp + 30 mg baobab | 1.5 | 162 | 70.53 |
| 190705 4/CSQU | GR 2000 mg / amp + 50 mg baobab | 2.5 | 98 | 41.37 |
| 190705 5/CSQU | GR 2000 mg / amp + 70 mg baobab | 3.5 | 100 | 42.30 |
| 190705 6/CSQU | GR 2000 mg / amp + 100 mg baobab | 5 | 101 | 42.76 |

Les valeurs nettement hors champ d'application de la norme apparaissent soulignées.

La viscosité cinématique est calculée selon la formule associée à la coupe d'écoulement n°3 (cf. NF EN ISO 2431 [7], 5.1.5 tableau 1)

Si l'on prend en compte toutes les mesures qu'il a été possible de réaliser, la différence de viscosité cinématique est telle que cela écrase les courbes et notamment celle de la gelée royale 1000 mg/10 ml. Pour cette raison et afin de faciliter la comparaison des formules et la compréhension du phénomène induit par l'ajout de poudre de pulpe de fruit de baobab, deux résultats ont été écartés de cette traduction graphique (Gelée royale 1500 mg sans poudre de baobab (19 23 05 3/CSQU) avec viscosité cinématique de 903.62 mm2/s et Gelée royale 2000 mg avec 1 % de poudre de baobab (19 07 05 2/CSQU) avec viscosité cinématique de 192.69 mm2/s). L'on obtient alors la représentation graphique présentée en figure 1.

Sur la figure 1, l'on observe que, pour les trois concentrations de gelée royale, les courbes présentent un point d'inflexion à partir duquel l'influence de la poudre de pulpe de fruit de baobab est optimale. Ces points d'inflexion se trouvent être parfaitement alignés : Pour une quantité de gelée royale pasteurisée introduite dans un volume donné (10 ml) la proportion relative de poudre de pulpe de fruit de baobab x par rapport la gelée royale à introduire est fonction de la viscosité cinématique critique y à atteindre.

Cette proportion est donnée par l'équation $y = 22.248 x - 14.222$ où y est la viscosité cinématique critique et x la proportion de poudre de pulpe de fruit de baobab (en masse) par rapport à la quantité de gelée royale.

Exemple 5 : Etude préliminaire concernant le mode d'action de la poudre de pulpe de fruit de baobab en tant qu'anti-gélifiant

Afin de déterminer si l'action de la poudre de pulpe de fruit de baobab sur la gelée royale est plutôt mécanique (en cassant les liaisons entre protéines) ou plutôt physique (spatiale) par occupation d'un espace empêchant la liaison entre protéines, la poudre de baobab a été ajoutée à 2000 mg de gelée royale pour 10 ml d'eau déjà chauffée à 60°C pendant 30 minutes.

Le mode opératoire en termes de mesure de viscosité cinématique est *mutatis mutandis* celui de l'exemple 4.

Les résultats sont présentés dans le tableau 16 infra :

Tableau 16

| Désignation | Proportion baobab (%) | Durée écoulement (s) | Viscosité cinématique (mm$^2$/s) |
|---|---|---|---|
| GR 2000 mg / amp chauffage 30 min à 60°C | 0 | 103 | 43.69 |
| GR 2000 mg / amp + baobab | 0.1 | 47 | 16.57 |
| GR 2000 mg / amp chauffage 30 min à 60°C puis ajout baobab | 0.1 | 46 | 16.03 |

La poudre de pulpe de fruit de baobab semble bien avoir une action mécanique sur le réseau qui se forme après chauffage. En effet, si l'action de la poudre de pulpe de fruit de baobab avait été par occupation physique de l'espace empêchant la polymérisation, l'ajout de cette poudre sur la gelée royale chauffée n'aurait dû avoir aucun effet : or, l'on retrouve la même durée d'écoulement que la gelée royale à laquelle l'on a ajouté 0,1% de poudre de pulpe de fruit de baobab et que l'on a chauffée ensuite.

Exemple 6 : Evaluation de l'effet « anti-gélifiant » sur la gelée royale d'un autre ingrédient contenant des fibres : le fibrulose

6.1 Objectif

Les inventeurs ont voulu tester la capacité d'un autre ingrédient contenant des fibres, le fibrulose, à éviter le phénomène de gélification de la gelée royale lors d'un traitement thermique.

Le fibrulose est une fibre soluble végétale extraite de la racine de chicorée.

6.2 Mode opératoire

Dans un bêcher, incorporer la quantité d'eau nécessaire, sous agitation, solubiliser le FIBRULOSE dans l'eau puis, après solubilisation de celui-ci, ajouter 1500 mg de gelée royale (Gelée Royale Bio+). Mélanger le tout pendant 10 minutes. Puis mettre le mélange en ampoules. Ensuite, réaliser une pasteurisation à environ 90°C pendant environ 60 minutes.

Les compositions suivantes ont été testées :

Tableau 17

| MATIERES PREMIERES | MG | % |
|---|---|---|
| GELEE ROYALE BIO + | 1500 | 9,091 % |
| PILOTE FIBRULOSE/DM | 15 | 0,091% |
| EAU DE VILLE | 14985 | 90,818% |
| TOTAL | 16500,0000 mg | 100,000% |
| (ampoule 15 ml) | | |

Tableau 18

| MATIERES PREMIERES | MG | % |
|---|---|---|
| GELEE ROYALE BIO + | 1500 | 9,091% |
| PILOTE FIBRULOSE/DM | 50 | 0,303% |
| EAU DE VILLE | 14950 | 90,606% |
| TOTAL | 16500,0000 mg | 100,000% |
| (ampoule 15 ml) | | |

Tableau 19

| MATIERES PREMIERES | MG | % |
|---|---|---|
| GELEE ROYALE BIO + | 1500 | 9,091 % |
| PILOTE FIBRULOSE/DM | 100 | 0,606% |
| EAU DE VILLE | 14900 | 90,303% |
| TOTAL | 16500,0000 mg | 100,000% |
| (ampoule 15 ml) | | |

6.3 Résultats

Concernant la composition présentée au sein du tableau 17 supra, l'on constate une gélification importante, même après homogénéisation. L'ampoule se vide mais la restitution du mélange est incomplète.

Concernant la composition présentée au sein du tableau 18 supra, l'on constate :

- une gélification plus importante ;
- après homogénéisation, aspect un peu plus fluide mais reste assez gélatineux, et
- un écoulement assez difficile et une restitution du mélange est incomplète.

En dernier lieu, concernant la composition présentée au sein du tableau 19 supra, l'on constate :

- une gélification beaucoup plus importante ;
- après homogénéisation, un aspect très peu fluide, assez gélatineux, et
- un écoulement difficile, et la restitution du mélange est incomplète.

6.4 Conclusion

En conclusion, la stabilisation de la gelée royale, en particulier via un effet anti-gélifiant lorsqu'elle est soumise à un traitement thermique apparaît être une propriété propre et spécifique de la poudre de pulpe de fruit de baobab.

Exemple 7 : Contrôle de la qualité du produit

7.1 Objectif

L'objectif du présent exemple consiste à vérifier, sur certaines compositions testées en viscosité (cf. exemple 3 supra), l'absence d'impact de la poudre de pulpe de fruit de baobab sur la teneur en 10-2-HDA (acide 10-hydroxy-2-décénoïque ; n°CAS : 14113-05-4), et donc sur la qualité de la gelée royale obtenue après traitement thermique (le 10-2-HDA étant un marqueur de qualité de la gelée royale).

7.2 Mode opératoire

7.2.1. Préparation des solutions :

Phase mobile :

Dans une éprouvette de 1000 ml, introduire 500 ml de méthanol qualité HPLC et 500 ml d'eau purifiée acidifiée à pH 2.50 avec de l'acide phosphorique 85 %. Mélanger puis filtrer sous vide.

Solution à examiner :

Dans un erlenmeyer, mélanger le contenu de 3 ampoules.
Dans une fiole jaugée de 50,0 ml, introduire une quantité exactement pesée voisine de 10,00 g du mélange des contenus des ampoules et ajouter 40 ml de méthanol analytique. Placer sous agitation magnétique 10 minutes puis placer la solution au bain à ultrasons pendant 10 minutes. Laisser revenir à température ambiante la solution avant de compléter au volume avec le même solvant. Homogénéiser et laisser décanter quelques minutes avant de prélever 2,0 ml du surnageant et compléter à 25,0 ml avec la phase mobile. Homogénéiser et filtrer sur membrane PVDF 0,22 $\mu$m avant d'injecter.

Solution de référence :

Dans une fiole jaugée de 25,0 ml, introduire 6,00 mg de 10-2-HDA (TCI-référence H1337) exactement pesé et 8 ml de méthanol analytique. Placer sous agitation magnétique pendant 10 minutes puis placer la solution au bain à ultrasons pendant 10 minutes. Laisser revenir à température ambiante la solution avant de compléter au volume avec le même solvant. Homogénéiser et prélever 1,0 ml de la solution obtenue et compléter à 25,0 ml avec la phase mobile. Homogénéiser et filtrer sur membrane PVDF 0,22 $\mu$m avant d'injecter.

7.2.2. Conditions chromatographiques :

Colonne (équipée d'un pré-filtre en ligne) : type Zorbax SB

- dimensions : l = 50 mm, Ø = 4,6 mm,
- phase stationnaire : C18, taille des particules = 1,8 $\mu$m,
- température : 25 °C

Phase mobile en mode isocratique : MeOH / Eau purifiée à pH=2.50 H3PO4 (50/50) (V/V) Débit : 0,55 ml/min
Détection : spectrophotomètre UV/Visible à 210 nm (BW = 4, Réf = Off) Injection : 10 $\mu$l
Temps d'analyse : 8 min

7.2.3. Calculs :

Calculer la teneur en 10-2-HDA (en mg/ampoule, *m/m)* à l'aide de l'expression suivante :

$$T\,(mg/amp) = \frac{Ae \times mt \times P \times Ve \times Mthe}{At \times me \times Vt}$$

où :

Ae : Surface du pic correspondant au 10-2-HDA dans le chromatogramme obtenu avec la solution à examiner

At : Surface du pic correspondant au 10-2-HDA dans le chromatogramme obtenu avec la solution de référence

me : Prise d'essai de l'échantillon, en mg

mt : Prise d'essai de la substance de référence, en mg

P : Pureté de la substance de référence, en pour cent (ex : 0,98)

Ve : Volume de dilution de l'échantillon, en ml (625)

Vt : Volume de dilution de la substance de référence, en ml (625)

Mthe : Masse théorique d'une ampoule, en mg (10000 x densité)

7.3 Résultats

La matière première gelée royale ayant servi à la fabrication des compositions testées a été analysée pour déterminer la valeur de référence en 10-2-HDA attendue pour chaque formule :

Tableau 20

| Lot | Désignation | Teneur en 10-2-HDA (en % m/m) | Teneur théorique en 10-2-HDA (en mg/ampoule) | |
|---|---|---|---|---|
| | | | 1000 mg GR | 2000 mg GR |
| 1180006893 | Gelée Royale Bio+ | 1,49 | 14,9 | 29,8 |

Les résultats obtenus pour les différentes compositions testées sont présentés dans le tableau 21 infra :

Tableau 21

| Lot | Désignation | Teneur en 10-2-HDA (en mg/ampoule) | Recouvrement / théorie |
|---|---|---|---|
| 19 06 05 2 / CSQU | Amp. GR 1000 mg + 10 mg poudre baobab | 14,0 | 94,0 % |
| 19 06 05 4 / CSQU | Amp. GR 1000 mg + 20 mg poudre baobab | 14,2 | 95,3 % |
| 19 06 05 5 / CSQU | Amp. GR 1000 mg + 30 mg poudre baobab | 14,5 | 97,3 % |
| 19 07 05 1 / CSQU | Amp. GR 2000 mg + 10 mg poudre baobab | 28,8 | 96,6 % |
| 19 07 05 2 / CSQU | Amp. GR 2000 mg + 20 mg poudre baobab | 28,9 | 97,0 % |
| 19 07 05 3 / CSQU | Amp. GR 2000 mg + 30 mg poudre baobab | 28,0 | 94,0 % |

(suite)

| Lot | Désignation | Teneur en 10-2-HDA (en mg/ ampoule) | Recouvrement / théorie |
|---|---|---|---|
| 19 07 05 6 / CSQU | Amp. GR 2000 mg + 100 mg poudre baobab | 33,7 | 113,1 % |

D'après les données mentionnées au sein du tableau 21 il apparaît très clairement que la quantité de 10-2-HDA - et donc la qualité de la gelée royale - n'est pas impactée de manière significative dans ces différentes formulations.

Il peut donc être conclu que la poudre de pulpe de fruit de baobab permet de stabiliser la gelée royale, en particulier en prévenant, limitant et/ou inhibant le phénomène de gélification se produisant lorsque ladite gelée royale est soumise à un traitement thermique (par exemple de type pasteurisation ou stérilisation), tout en préservant la qualité (notamment la qualité nutritive) de la gelée royale (et les propriétés physico-chimiques de celle-ci ; cf. exemple 3 supra).

**Références bibliographiques**

**[1]** KHAZAEI, M et al. «New Findings on biological actions and clinical applications of royal jelly: A Review». Journal of dietary supplements, (2017). 1-19. Disponible sur: http://www.manukahealth.co.nz/media/1338/research-paper-new-findings-on-biologicalactions-and-clinical-applications-of-royal-jelly-a-review-2017.pdf

**[2]** FRATINI, F et al. «Royal Jelly: An ancient remedy with remarkable antibacterial properties». Microbiological Research, (2016). 192: 130-141. Disponible sur : https://www.sciencedirect.com/science/article/pii/S0944501316300830

**[3]** Norme « NF ISO 12824». AFNOR (26 novembre 2016). Indice de classement : V 36-001. « Gelée royale - spécifications».

**[4]** JACKSON, S. «Baobab: The Tree of Life - An Ethnopharmacological Review». HerbalGram. American Botanical Council, (2015).108: 42-53.

**[5]** DIOP, A et al. «Le baobab africain (Adansonia digitata L.) : principales caractéristiques et utilisations ». Fruits, (2006). 61 (1), pp.55-69. Disponible sur: https://hal.archivesouvertes. fr/hal-01517201/document

**[6]** Direction générale de l'alimentation ; Sous-direction de la sécurité sanitaire des aliments Ordre de service d'action « Instruction technique DGAL/SDSSA/2015-364 », 06 octobre 2015 (publiée le 13 octobre 2015). Disponible sur: https://info.agriculture.gouv.fr/gedei/site/boagri/instruction-2015-364

**[7]** Norme « NF EN ISO 2431». AFNOR (décembre 2011). Indice de classement : T 30-070. « Peintures et vernis Détermination du temps d'écoulement au moyen de coupes d'écoulement».

**Revendications**

1. Utilisation d'au moins une préparation à base de pulpe de fruit de baobab pour stabiliser une préparation comprenant de la gelée royale.

2. Utilisation selon la revendication précédente, dans laquelle l'action de stabiliser comprend, consiste essentiellement à, ou consiste à :

   - stabiliser dans le temps ladite préparation comprenant de la gelée royale, et/ou
   - prévenir, limiter et/ou inhiber la gélification de ladite préparation comprenant de la gelée royale, de préférence lorsque ladite préparation comprenant de la gelée royale est soumise à un traitement thermique tel qu'une pasteurisation ou une stérilisation, préférablement à une pasteurisation, avantageusement à une température comprise entre environ 60°C et environ 99°C, et de manière préférée à une température comprise entre environ 75°C et environ 95°C.

**3.** Utilisation selon la revendication 1 ou 2, dans laquelle la préparation comprenant de la gelée royale est dépourvue de conservateur et avantageusement également dépourvue de composé de synthèse.

**4.** Utilisation selon l'une des revendications précédentes, dans laquelle la gelée royale est sélectionnée parmi la gelée royale fraîche, la gelée royale décongelée, la gelée royale lyophilisée, la gelée royale atomisée.

**5.** Utilisation selon l'une des revendications précédentes, dans laquelle la préparation comprenant de la gelée royale consiste en :

i) la gelée royale, ou

ii) une préparation comprenant, consistant essentiellement en, ou consistant en, un mélange de gelée royale et d'eau, de préférence dans un rapport en masse gelée royale/eau compris entre 5/200 et 1/2, préférablement entre 5/100 et 2/5, avantageusement entre 2/25 et 3/10 ; de préférence ladite préparation ii) étant sous forme de solution buvable, avantageusement adaptée pour être conditionnée dans un contenant à usage unique clos, de préférence de manière hermétique, tel qu'un sachet unidose, un stick unidose, une flaconnette, une ampoule bouteille unidose, ou une ampoule, de préférence une ampoule, préférablement une ampoule à extrémité(s) cassable(s), avantageusement une ampoule à extrémités cassables telle qu'une ampoule en verre, par exemple d'une contenance de 10 ml.

**6.** Utilisation selon l'une des revendications précédentes, dans laquelle ladite au moins une préparation à base de pulpe de fruit de baobab est sélectionnée parmi : poudre de pulpe de fruit de baobab et/ou extrait de pulpe de fruit de baobab ; ladite au moins une préparation à base de pulpe de fruit de baobab étant avantageusement de la poudre de pulpe de fruit de baobab.

**7.** Utilisation selon l'une des revendications précédentes, dans laquelle la masse de ladite au moins une préparation à base de pulpe de fruit de baobab représente au moins 0,5%, de préférence au moins 1%, préférablement au moins 1,3%, avantageusement au moins 1,5%, de manière préférée au moins 1,8%, et de manière particulièrement préférée au moins 2%, de la masse de gelée royale.

**8.** Utilisation selon l'une des revendications 1 à 6, dans laquelle au moins x% de ladite au moins une préparation à base de pulpe de fruit de baobab, défini(s) par rapport à la masse de gelée royale, est/sont mélangé(s) à la préparation comprenant de la gelée royale pour atteindre une viscosité cinématique critique prédéterminée y, à partir de laquelle l'écoulement du mélange est optimal, et dans laquelle ledit/lesdits x% est/sont déterminé(s) par la relation suivante :

$$x\% = \frac{y + 14{,}222}{22{,}248}$$

**9.** Utilisation selon l'une des revendications 1 à 6, dans laquelle ladite préparation comprenant de la gelée royale comprend, consiste essentiellement en, ou consiste en :

- 1000 mg de gelée royale et ladite au moins une préparation à base de pulpe de fruit de baobab est utilisée à raison d'au moins 0,5% en masse, de préférence d'au moins 1% en masse, par rapport à la masse de la gelée royale, ou
- 1500 mg de gelée royale et ladite au moins une préparation à base de pulpe de fruit de baobab est utilisée à raison d'au moins 0,5% en masse, de préférence d'au moins 1% en masse, préférablement d'au moins 1,33% en masse, par rapport à la masse de la gelée royale, ou
- 2000 mg de gelée royale et ladite au moins une préparation à base de pulpe de fruit de baobab est utilisée à raison d'au moins 1% en masse, de préférence d'au moins 1,5% en masse, préférablement d'au moins 2% en masse, et avantageusement d'au moins 2,5% en masse, par rapport à la masse de la gelée royale.

**10.** Composition comprenant, consistant essentiellement en, ou consistant en :

i) au moins une préparation à base de pulpe de fruit de baobab telle que définie dans l'une des revendications précédentes, et

ii.1) une préparation comprenant de la gelée royale telle que définie dans l'une des revendications précédentes, la masse de ladite au moins une préparation à base de pulpe de fruit de baobab représentant plus de 1,5%, de préférence au moins 1,8%, préférablement au moins 2%, avantageusement au moins 3%, de la masse de gelée royale, ou

ii.2) une préparation comprenant de la gelée royale comprenant, consistant essentiellement en, ou consistant en :

- 1500 mg de gelée royale, et ladite au moins une préparation à base de pulpe de fruit de baobab représentant au moins 0,5% en masse, de préférence au moins 1% en masse, préférablement au moins 1,33% en masse, par rapport à la masse de la gelée royale, ou
- 2000 mg de gelée royale, et ladite au moins une préparation à base de pulpe de fruit de baobab représentant au moins 1% en masse, de préférence au moins 1,5% en masse, préférablement au moins 2% en masse, et avantageusement au moins 2,5% en masse, par rapport à la masse de la gelée royale.

de préférence ladite composition étant dépourvue de conservateur et avantageusement également dépourvue de composé de synthèse.

11. Contenant à usage unique clos, de préférence de manière hermétique, tel qu'un sachet unidose, un stick unidose, une flaconnette, ou une ampoule à extrémité(s) cassable(s), ledit contenant comprenant la composition selon la revendication précédente, ledit contenant étant de préférence une ampoule à extrémités cassables en plastique ou en verre, de préférence en verre, par exemple d'une contenance de 10 ou 15 ml.

12. Procédé thermique de stabilisation et de décontamination d'une préparation comprenant de la gelée royale telle que définie dans l'une des revendications précédentes, ledit procédé comprenant les étapes suivantes :

a) obtenir ladite préparation comprenant de la gelée royale, de préférence comprenant, consistant essentiellement en, ou consistant en, un mélange de gelée royale et d'eau, de préférence dans un rapport en masse gelée royale/eau compris entre 5/200 et 1/2, préférablement entre 5/100 et 2/5, avantageusement entre 2/25 et 3/10,
b) introduire ladite préparation au sein d'un récipient, de préférence obturable, avantageusement de manière hermétique, ledit récipient étant adapté pour réaliser un traitement thermique de décontamination tel qu'un traitement de type pasteurisation ou stérilisation, préférablement de type pasteurisation,
c) à l'étape b), ou de préférence avant ladite étape b), mélanger ladite préparation comprenant de la gelée royale avec ladite au moins une préparation à base de pulpe de fruit de baobab telle que définie dans l'une des revendications précédentes,
d) postérieurement à l'étape b), effectuer, au sein dudit récipient, un traitement thermique de type pasteurisation ou stérilisation, de préférence de type pasteurisation, avantageusement à une température comprise entre environ 60°C et environ 99°C, préférablement à une température comprise entre environ 75°C et environ 95°C.

13. Procédé selon la revendication 12, dans lequel, à l'étape c), le mélange est réalisé dans un rapport en masse préparation à base de pulpe de fruit de baobab/gelée royale d'au moins 5/1000, de préférence d'au moins 1/100, préférablement d'au moins 1/75, avantageusement d'au moins 3/200, de manière préférée d'au moins 1,8/100, et de manière particulièrement préférée d'au moins 1/50.

14. Procédé selon la revendication 12, dans lequel au moins x% de ladite au moins une préparation à base de pulpe de fruit de baobab, défini(s) par rapport à la masse de gelée royale, est/sont mélangé(s), à l'étape c), avec ladite préparation comprenant de la gelée royale pour atteindre une viscosité cinématique critique prédéterminée y, à partir de laquelle l'écoulement du mélange est optimal, ledit/lesdits x% étant déterminé(s) par la relation suivante :

$$x\% = \frac{y + 14{,}222}{22{,}248}$$

15. Procédé selon la revendication 12, dans lequel :

- ladite préparation comprenant de la gelée royale comprend, consiste essentiellement en, ou consiste en, 1000 mg de gelée royale et, à l'étape c), le mélange est réalisé dans un rapport en masse préparation à base de pulpe de fruit de baobab/gelée royale d'au moins 5/1000, de préférence d'au moins 1/100, ou
- ladite préparation comprenant de la gelée royale comprend, consiste essentiellement en, ou consiste en, 1500 mg de gelée royale et, à l'étape c), le mélange est réalisé dans un rapport en masse préparation à base de pulpe de fruit de baobab/gelée royale d'au moins 5/1000, de préférence d'au moins 1/100, préférablement d'au moins 1/75, ou

- ladite préparation comprenant de la gelée royale comprend, consiste essentiellement en, ou consiste en, 2000 mg de gelée royale et, à l'étape c), le mélange est réalisé dans un rapport en masse préparation à base de pulpe de fruit de baobab/gelée royale d'au moins 1/100, de préférence d'au moins 3/200, préférablement d'au moins 1/50, et avantageusement d'au moins 5/200.

**Patentansprüche**

1. Eine Verwendung mindestens einer Zubereitung auf Basis von Baobab-Fruchtfleisch zur Stabilisierung einer Gelée royale beinhaltenden Zubereitung.

2. Verwendung gemäß dem vorhergehenden Anspruch, wobei die Stabilisierungsmaßnahme Folgendes beinhaltet, im Wesentlichen daraus besteht oder daraus besteht:

   - rechtzeitiges Stabilisieren der Gelée royale beinhaltenden Zubereitung, und/oder
   - Verhindern, Begrenzen und/oder Hemmen der Gelbildung der Gelée royale beinhaltenden Zubereitung, bevorzugt, wenn die Gelée royale beinhaltende Zubereitung einer Wärmebehandlung wie einer Pasteurisierung oder einer Sterilisierung, vorzugsweise einer Pasteurisierung, vorteilhafterweise bei einer Temperatur zwischen etwa 60 °C und etwa 99 °C, und auf bevorzugte Weise bei einer Temperatur zwischen etwa 75 °C und etwa 95 °C, unterzogen wird.

3. Verwendung gemäß Anspruch 1 oder 2, wobei die Gelée royale beinhaltende Zubereitung frei von Konservierungsmitteln und vorteilhafterweise auch frei von synthetischer Verbindung ist.

4. Verwendung gemäß einem der vorhergehenden Ansprüche, wobei das Gelée royale ausgewählt ist aus frischem Gelée royale, aufgetautem Gelée royale, gefriergetrocknetem Gelée royale, zerstäubtem Gelée royale.

5. Verwendung gemäß einem der vorhergehenden Ansprüche, wobei die Gelée royale beinhaltende Zubereitung aus Folgendem besteht:

   i) dem Gelée royale oder
   ii) einer Zubereitung, die eine Mischung aus Gelée royale und Wasser beinhaltet, im Wesentlichen daraus besteht oder daraus besteht, bevorzugt in einem Massenverhältnis von Gelée royale zu Wasser zwischen 5 : 200 und 1 : 2, vorzugsweise zwischen 5 : 100 und 2 : 5, vorteilhafterweise zwischen 2 : 25 und 3 : 10; wobei die Zubereitung ii) bevorzugt in Form einer trinkbaren Lösung vorliegt, die vorteilhafterweise angepasst ist, um in einem geschlossenen Einwegbehälter verpackt zu werden, bevorzugt hermetisch, wie ein Einzeldosisbeutel, ein Einzeldosisstick, ein Fläschchen, eine Einzeldosisampullenflasche oder eine Ampulle, bevorzugt eine Ampulle, vorzugsweise eine Ampulle mit abbrechbarem/n Ende/n, vorteilhafterweise eine Ampulle mit abbrechbarem/n Ende/n wie eine Glasampulle, beispielsweise mit einem Fassungsvermögen von 10 ml.

6. Verwendung gemäß einem der vorhergehenden Ansprüche, wobei die mindestens eine Zubereitung auf Basis von Baobab-Fruchtfleisch ausgewählt ist aus Folgendem: Baobab-Fruchtfleischpulver und/oder Baobab-Fruchtfleischextrakt; wobei die mindestens eine Zubereitung auf Basis von Baobab-Fruchtfleisch vorteilhafterweise Baobab-Fruchtfleischpulver ist.

7. Verwendung gemäß einem der vorhergehenden Ansprüche, wobei die Masse der mindestens einen Zubereitung auf Basis von Baobab-Fruchtfleisch mindestens 0,5 %, bevorzugt mindestens 1 %, vorzugsweise mindestens 1,3 %, vorteilhafterweise mindestens 1,5 %, auf bevorzugte Weise mindestens 1,8 % und auf besonders bevorzugte Weise mindestens 2 % der Masse an Gelée royale ausmacht.

8. Verwendung gemäß einem der Ansprüche 1 bis 6, wobei mindestens x % der mindestens einen Zubereitung auf Basis von Baobab-Fruchtfleisch, definiert in Bezug auf die Masse an Gelée royale, mit der Gelée royale beinhaltenden Zubereitung gemischt wird/werden, um eine vorgegebene kritische kinematische Viskosität y zu erreichen, ab welcher das Fließen der Mischung optimal ist, und wobei x % durch die folgende Beziehung bestimmt wird/werden:

$$x\,\% = \frac{y + 14{,}222}{22{,}248}$$

9. Verwendung gemäß einem der Ansprüche 1 bis 6, wobei die Gelée royale beinhaltende Zubereitung Folgendes beinhaltet, im Wesentlichen daraus besteht oder daraus besteht:

- 1 000 mg Gelée royale, und wobei die mindestens eine Zubereitung auf Basis von Baobab-Fruchtfleisch in einer Menge von mindestens 0,5 Massen-%, bevorzugt mindestens 1 Massen-%, bezogen auf die Masse des Gelée royale, verwendet wird, oder
- 1 500 mg Gelée royale, und wobei die mindestens eine Zubereitung auf Basis von Baobab-Fruchtfleisch in einer Menge von mindestens 0,5 Massen-%, bevorzugt mindestens 1 Massen-%, vorzugsweise mindestens 1,33 Massen-%, bezogen auf die Masse des Gelée royale, verwendet wird, oder
- 2 000 mg Gelée royale, und wobei die mindestens eine Zubereitung auf Basis von Baobab-Fruchtfleisch in einer Menge von mindestens 1 Massen-%, bevorzugt mindestens 1,5 Massen-%, vorzugsweise mindestens 2 Massen-% und vorteilhafterweise mindestens 2,5 Massen-%, bezogen auf die Masse des Gelée royale, verwendet wird.

10. Eine Zusammensetzung, die Folgendes beinhaltet, im Wesentlichen daraus besteht oder daraus besteht:

i) mindestens eine/einer Zubereitung auf Basis von Baobab-Fruchtfleisch wie in einem der vorhergehenden Ansprüche definiert, und
ii.1) eine/einer Zubereitung, die Gelée royale wie in einem der vorhergehenden Ansprüche definiert beinhaltet, wobei die Masse der mindestens einen Zubereitung auf Basis von Baobab-Fruchtfleisch mehr als 1,5 %, bevorzugt mindestens 1,8 %, vorzugsweise mindestens 2 %, vorteilhafterweise mindestens 3 % der Masse von Gelée royale ausmacht, oder
ii.2) eine/einer Gelée royale beinhaltende/n Zubereitung, die Folgendes beinhaltet, im Wesentlichen daraus besteht oder daraus besteht:

- 1 500 mg Gelée royale, und wobei die mindestens eine Zubereitung auf Basis von Baobab-Fruchtfleisch mindestens 0,5 Massen-%, bevorzugt mindestens 1 Massen-%, vorzugsweise mindestens 1,33 Massen-%, bezogen auf die Masse des Gelée royale, ausmacht, oder
- 2 000 mg Gelée royale, und wobei die mindestens eine Zubereitung auf Basis von Baobab-Fruchtfleisch mindestens 1 Massen-%, bevorzugt mindestens 1,5 Massen-%, vorzugsweise mindestens 2 Massen-% und vorteilhafterweise mindestens 2,5 Massen-%, bezogen auf die Masse des Gelée royale, ausmacht,

wobei die Zusammensetzung bevorzugt frei von Konservierungsmitteln und vorteilhafterweise auch frei von synthetischer Verbindung ist.

11. Ein verschlossener, bevorzugt auf hermetische Weise, Einwegbehälter wie ein Einzeldosisbeutel, ein Einzeldosisstick, ein Fläschchen oder eine Ampulle mit abbrechbarem/n Ende/n, wobei der Behälter die Zusammensetzung gemäß dem vorhergehenden Anspruch beinhaltet, wobei der Behälter bevorzugt eine Ampulle mit abbrechbaren Enden aus Kunststoff oder Glas, bevorzugt aus Glas, beispielsweise mit einem Fassungsvermögen von 10 oder 15 ml, ist.

12. Ein thermisches Verfahren zur Stabilisierung und Dekontaminierung einer Gelée royale beinhaltenden Zubereitung wie in einem der vorhergehenden Ansprüche definiert, wobei das Verfahren die folgenden Schritte beinhaltet:

a) Erhalten der Gelée royale beinhaltenden Zubereitung, die bevorzugt eine Mischung aus Gelée royale und Wasser beinhaltet, im Wesentlichen daraus besteht oder daraus besteht:, bevorzugt in einem Massenverhältnis von Gelée royale zu Wasser zwischen 5 : 200 und 1 : 2, vorzugsweise zwischen 5 : 100 und 2 : 5, vorteilhafterweise zwischen 2 : 25 und 3 : 10,
b) Einbringen der Zubereitung in ein bevorzugt verschließbares, vorteilhafterweise auf hermetische Weise, Behältnis, wobei das Behältnis angepasst ist, um eine thermische Dekontaminationsbehandlung wie eine Behandlung vom Pasteurisierungs- oder Sterilisierungstyp, vorzugsweise vom Pasteurisierungstyp, durchzuführen,
c) in Schritt b) oder bevorzugt vor dem Schritt b), Mischen der Gelée royale beinhaltenden Zubereitung mit der mindestens einen Zubereitung auf Basis von Baobab-Fruchtfleisch wie in einem der vorhergehenden Ansprüche definiert,
d) nach Schritt b), Vornehmen, in dem Behältnis, einer Wärmebehandlung vom Pasteurisierungs- oder Sterilisierungstyp, bevorzugt vom Pasteurisierungstyp, vorteilhafterweise bei einer Temperatur zwischen etwa 60 °C und etwa 99 °C, vorzugsweise bei einer Temperatur zwischen etwa 75 °C und etwa 95 °C.

**13.** Verfahren gemäß Anspruch 12, wobei in Schritt c) das Mischen in einem Massenverhältnis der Zubereitung auf Basis von Baobab-Fruchtfleisch zu Gelée royale von mindestens 5 : 1 000, bevorzugt von mindestens 1 : 100, vorzugsweise von mindestens 1 : 75, vorteilhafterweise von mindestens 3 : 200, auf bevorzugte Weise von mindestens 1,8 : 100 und auf besonders bevorzugte Weise von mindestens 1 : 50 durchgeführt wird.

**14.** Verfahren gemäß Anspruch 12, wobei mindestens x % der mindestens einen Zubereitung auf Basis von Baobab-Fruchtfleisch, definiert in Bezug auf die Masse an Gelée royale, in Schritt c) mit der Gelée royale beinhaltenden Zubereitung gemischt wird/werden, um eine vorgegebene kritische kinematische Viskosität y zu erreichen, ab welcher das Fließen der Mischung optimal ist, wobei das/die x % durch die folgende Beziehung bestimmt wird/werden:

$$x\,\% \;=\; \frac{\text{y} + 14{,}222}{22{,}248}$$

**15.** Verfahren gemäß Anspruch 12, wobei:

- die Gelée royale beinhaltende Zubereitung 1 000 mg Gelée royale beinhaltet, im Wesentlichen daraus besteht oder daraus besteht, und wobei in Schritt c) das Mischen in einem Massenverhältnis der Zubereitung auf Basis von Baobab-Fruchtfleisch zu Gelée royale von mindestens 5 : 1 000, bevorzugt mindestens 1 : 100 durchgeführt wird, oder
- die Gelée royale beinhaltende Zubereitung 1 500 mg Gelée royale beinhaltet, im Wesentlichen daraus besteht oder daraus besteht, und wobei in Schritt c) das Mischen in einem Massenverhältnis der Zubereitung auf Basis von Baobab-Fruchtfleisch zu Gelée royale von mindestens 5 : 1 000, bevorzugt mindestens 1 : 100, vorzugsweise mindestens 1 : 75 durchgeführt wird, oder
- die Gelée royale beinhaltende Zubereitung 2 000 mg Gelée royale beinhaltet, im Wesentlichen daraus besteht oder daraus besteht, und wobei in Schritt c) das Mischen in einem Massenverhältnis der Zubereitung auf Basis von Baobab-Fruchtfleisch zu Gelée royale von mindestens 1 : 100, bevorzugt mindestens 3 : 200, vorzugsweise mindestens 1 : 50 und vorteilhafterweise mindestens 5 : 200 durchgeführt wird.

**Claims**

**1.** Use of at least one preparation based on baobab fruit pulp for stabilising a preparation comprising royal jelly.

**2.** The use according to the preceding claim, wherein the stabilising action comprises, consists essentially in, or consists in:

- stabilising, overtime, said preparation comprising royal jelly, and/or
- preventing, limiting and/or inhibiting the gelification of said preparation comprising royal jelly, preferably when said preparation comprising royal jelly is subjected to a thermal treatment such as pasteurisation or sterilisation, preferably pasteurisation, advantageously at a temperature of between approximately 60°C and approximately 99°C, and in a preferred manner at a temperature of between approximately 75°C and approximately 95°C.

**3.** The use according to Claim 1 or 2, wherein the preparation comprising royal jelly is free of preservative and advantageously also free of synthetic compound.

**4.** The use according to one of the preceding claims, wherein the royal jelly is selected from fresh royal jelly, defrosted royal jelly, freeze-dried royal jelly, atomised royal jelly.

**5.** The use according to one of the preceding claims, wherein the preparation comprising royal jelly consists of:

i) royal jelly, or
ii) a preparation comprising, consisting essentially of, or consisting of, a mixture of royal jelly and water, preferably in a royal jelly:water mass ratio of between 5:200 and 1:2, preferably between 5:100 and 2:5, advantageously between 2:25 and 3:10; preferably said preparation ii) being in the form of a drinkable solution, advantageously suitable for being packaged in a sealed, preferably hermetically sealed, single-use container, such as a single-dose sachet, a single-dose stick, a vial, a single-dose ampoule bottle, or an ampoule, preferably an ampoule, preferably an ampoule with breakable end(s), advantageously an ampoule with breakable ends such as a glass ampoule, for example with a capacity of 10 ml.

6. The use according to one of the preceding claims, wherein said at least one preparation based on baobab fruit pulp is selected from: baobab fruit pulp powder and/or baobab fruit pulp extract; said at least one preparation based on baobab fruit pulp advantageously being baobab fruit pulp powder.

7. The use according to one of the preceding claims, wherein the mass of said at least one preparation based on baobab fruit pulp represents at least 0.5%, preferably at least 1%, preferably at least 1.3%, advantageously at least 1.5%, in a preferred manner at least 1.8%, and in a particularly preferred manner at least 2%, of the mass of royal jelly.

8. The use according to one of Claims 1 to 6, wherein at least x% of said at least one preparation based on baobab fruit pulp, defined with respect to the mass of royal jelly, is mixed with the preparation comprising royal jelly in order to reach a predetermined critical kinematic viscosity y, starting from which the flow of the mixture is optimal, and wherein said x% is determined by the following relationship:

$$x\% = \frac{y + 14.222}{22.248}$$

9. The use according to one of Claims 1 to 6, wherein said preparation comprising royal jelly comprises, consists essentially of, or consists of:

- 1000 mg of royal jelly and said at least one preparation based on baobab fruit pulp is used in an amount of at least 0.5% by mass, preferably at least 1% by mass, with respect to the mass of the royal jelly, or
- 1500 mg of royal jelly and said at least one preparation based on baobab fruit pulp is used in an amount of at least 0.5% by mass, preferably at least 1% by mass, preferably at least 1.33% by mass, with respect to the mass of the royal jelly, or
- 2000 mg of royal jelly and said at least one preparation based on baobab fruit pulp is used in an amount of at least 1% by mass, preferably at least 1,5% by mass, preferably at least 2% by mass, and advantageously at least 2.5% by mass, with respect to the mass of the royal jelly.

10. A composition comprising, consisting essentially of, or consisting of:

i) at least one preparation based on baobab fruit pulp as defined in one of the preceding claims, and
ii.1) a preparation comprising royal jelly as defined in one of the preceding claims, the mass of said at least one preparation based on baobab fruit pulp representing more than 1.5%, preferably at least 1,8%, preferably at least 2%, advantageously at least 3%, of the mass of royal jelly, or
ii.2) a preparation comprising royal jelly comprising, consisting essentially of, or consisting of:

- 1500 mg of royal jelly, and said at least one preparation based on baobab fruit pulp representing at least 0.5% by mass, preferably at least 1% by mass, preferably at least 1.33% by mass, with respect to the mass of the royal jelly, or
- 2000 mg of royal jelly, and said at least one preparation based on baobab fruit pulp representing at least 1% by mass, preferably at least 1,5% by mass, preferably at least 2% by mass, and advantageously at least 2.5% by mass, with respect to the mass of the royal jelly,

preferably said composition being free of preservative and advantageously also free of synthetic compound.

11. A sealed, preferably hermetically sealed, single-use container, such as a single-dose sachet, a single-dose stick, a vial, or an ampoule with breakable end(s), said container comprising the composition according to the preceding claim, said container preferably being a plastic or glass, preferably glass, ampoule with breakable ends, for example with a capacity of 10 or 15 ml.

12. A thermal process for stabilising and decontaminating a preparation comprising royal jelly as defined in one of the preceding claims, said process comprising the following steps:

a) obtaining said preparation comprising royal jelly, preferably comprising, consisting essentially of, or consisting of, a mixture of royal jelly and water, preferably in a royal jelly:water mass ratio of between 5:200 and 1:2, preferably between 5:100 and 2:5, advantageously between 2:25 and 3:10,

b) introducing said preparation into a container, preferably a closable container, advantageously a hermetically closable container, said container being suitable for performing a decontamination thermal treatment such as a pasteurisation-type or sterilisation-type treatment, preferably a pasteurisation-type treatment,

c) in step b), or preferably before said step b), mixing said preparation comprising royal jelly with said at least one preparation based on baobab fruit pulp as defined in one of the preceding claims,

d) after step b), carrying out, within said container, a thermal treatment of the pasteurisation or sterilisation type, preferably of the pasteurisation type, advantageously at a temperature of between approximately 60°C and approximately 99°C, preferably at a temperature of between approximately 75°C and approximately 95°C.

13. The process according to Claim 12, wherein, in step c), the mixture is made in a mass ratio of preparation based on baobab fruit pulp:royal jelly of at least 5:1000, preferably at least 1:100, preferably at least 1:75, advantageously at least 3:200, in a preferred manner at least 1.8:100 and in a particularly preferred manner at least 1:50.

14. The process according to Claim 12, wherein at least x% of said at least one preparation based on baobab fruit pulp, defined with respect to the mass of royal jelly, is mixed, in step c), with said preparation comprising royal jelly in order to reach a predetermined critical kinematic viscosity y, starting from which the flow of the mixture is optimal, said x% being determined by the following relationship:

$$x\% = \frac{y + 14.222}{22.248}$$

15. The process according to Claim 12, wherein:

- said preparation comprising royal jelly comprises, consists essentially of, or consists of, 1000 mg of royal jelly and, in step c), the mixture is made in a mass ratio of preparation based on baobab fruit pulp:royal jelly of at least 5:1000, preferably at least 1:100, or

- said preparation comprising royal jelly comprises, consists essentially of, or consists of, 1500 mg of royal jelly and, in step c), the mixture is made in a mass ratio of preparation based on baobab fruit pulp:royal jelly of at least 5:1000, preferably at least 1:100, preferably at least 1:75, or

- said preparation comprising royal jelly comprises, consists essentially of, or consists of, 2000 mg of royal jelly and, in step c), the mixture is made in a mass ratio of preparation based on baobab fruit pulp:royal jelly of at least 1:100, preferably at least 3:200, preferably at least 1:50, and advantageously at least 5:200.

*Fig. 1*

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2002176936 A

**Littérature non-brevet citée dans la description**

- *CHEMICAL ABSTRACTS,* 14113-05-4
- **KHAZAEI, M et al.** New Findings on biological actions and clinical applications of royal jelly: A Review. *Journal of dietary supplements,* 2017, 1-19, http://www.manukahealth.co.nz/media/1338/research-paper-new-findings-on-biologicalactions-and-clinical-applications-of-royal-jelly-a-review-2017.pdf
- **FRATINI, F et al.** Royal Jelly: An ancient remedy with remarkable antibacterial properties. *Microbiological Research,* 2016, vol. 192, 130-141, https://www.sciencedirect.com/science/article/pii/S0944501316300830
- Gelée royale - spécifications. *AFNOR,* 26 Novembre 2016
- **JACKSON, S.** Baobab: The Tree of Life - An Ethnopharmacological Review. *HerbalGram. American Botanical Council,* 2015, vol. 108, 42-53
- **DIOP, A et al.** Le baobab africain (Adansonia digitata L.) : principales caractéristiques et utilisations. *Fruits,* 2006, vol. 61 (1), 55-69, https://hal.archivesouvertes.fr/hal-01517201/document
- Direction générale de l'alimentation ; Sous-direction de la sécurité sanitaire des aliments Ordre de service d'action. *Instruction technique DGAL/SDS-SA/2015-364,* 06 Octobre 2015, https://info.agriculture.gouv.fr/gedei/site/boagri/instruction-2015-364
- Peintures et vernis Détermination du temps d'écoulement au moyen de coupes d'écoulement. *AFNOR,* Décembre 2011